# FASCICULE DE BREVET EUROPEEN

(11) **EP 2 297 148 B1**
(45) Date de publication et mention de la délivrance du brevet: **04.01.2012**
(21) Numéro de dépôt: 09761939.9
(22) Date de dépôt: 12.06.2009
(51) Int. Cl.: C07D 471/08, C07D 487/08, A61K 31/529, A61P 25/00, A61P 29/00, A61P 35/00

(54) **NOUVEAUX DERIVES DE (PIPERAZINYL PONTE)-1-ALCANONE ET LEUR UTILISATION COMME INHIBITEURS DE p75**
NEUE DERIVATE VON (VERBRÜCKTES PIPERAZINYL)-1-ALCANON UND DEREN VERWENDUNG ALS P75-INHIBITOREN
NOVEL DERIVATIVES OF (BRIDGED PIPERAZINYL)-1-ALCANONE AND USE THEREOF AS P75 INHIBITORS

(30) Priorité: 13.06.2008 FR 0803299
(43) Date de publication de la demande: 23.03.2011
(73) Titulaire: SANOFI, 75013 Paris (FR)
(72) Inventeur: BARONI, Marco, F-75013 Paris (FR); BONO, Françoise, F-75013 Paris (FR); DELBARY-GOSSART, Sandrine, F-75013 Paris (FR)
(74) Mandataire: Veinante, Aude
(86) Numéro de dépôt international: PCT/FR2009/051118
(87) Numéro de publication internationale: WO 2009/150388

(56) Documents cités:
- WO-A-03/104225
- WO-A-2005/054227
- WO-A-2005/054229

## Description

La présente invention a pour objet des dérivés de dérivés ((phényl)-3,6-dihydropyridin-1yl)-(pipérazinyl ponté)-1-alcanone et ((phényl)-2,5-dihydro-pyrrol-1yl)-(pipérazinyl ponté)-1-alcanone, leur préparation et leur application en thérapeutique.

Les composés selon la présente invention présentent une affinité pour le récepteur p75^{NTR} des neurotrophines.

Les neurotrophines appartiennent à une famille de protéines ayant notamment pour effet biologique la survie et la différenciation cellulaires.

Le récepteur p75^{NTR}, récepteur de toutes les neurotrophines, est une glycoprotéine transmembranaire de la famille du récepteur du facteur de nécrose tumorale (TNF, de l'anglais Tumor Necrosis Factor) (W. J. Friedman et L. A. Greene, Exp. Cell. Res., 1999,253, 131-142). Le récepteur p75^{NTR} est exprimé dans plusieurs types cellulaires, et plusieurs fonctions biologiques lui sont attribuées : d'une part, la modulation de l'affinité des neurotrophines pour les récepteurs tyrosine kinase (trk) ; d'autre part, en l'absence de trk, une induction d'un signal de mort cellulaire par apoptose. Par ailleurs, les précurseurs des neurotrophines, les proneurotrophines sont capables de se fixer sur p75^{NTR} avec une haute affinité, et sont considérés comme de puissants inducteurs de l'apoptose dépendant de p75^{NTR} dans les neurones et certaines lignées cellulaires.

Au niveau du système nerveux central, de nombreux travaux montrent que l'apoptose intervient dans plusieurs pathologies comme la sclérose latérale amyotrophique, la sclérose en plaques, les maladies d'Alzheimer, de Parkinson et d'Huntington et les maladies à prion. P75^{NTR} est également connu pour être surexprimé dans différents types de maladies neurodégénératives comme la maladie d'Alzheimer et la sclérose latérale amyotrophique (ALS) (Longo F. M. et al., Curr. Alzheimer Res. 2007;4: 503-506; Lowry K.S. et al., Amyotroph. Lateral. Scler. Other. Motor. Neuron. Disord. 2001; 2:127-34).

Des résultats suggèrent que p75^{NTR} peut jouer un rôle prépondérant dans les mécanismes conduisant à la mort neuronale par apoptose post-ischémie (P. P. Roux et al., J. Neurosci., 1999, 19, 6887- 6896).

Des résultats (V. Della-Bianca et al., J. Biol. Chem., 2001, 276 : 38929-33), (S. Rabizadeh et al., Proc. Natl. Acad. Sci. USA, 1994,91, 10703-10706) supportent l'hypothèse selon laquelle p75^{NTR} jouerait un rôle important dans la mort neuronale induite par la protéine prion infectieuse (encéphalopathie spongiforme transmissible) ou par la protéine beta Amyloide (maladie d'Alzheimer).

Le récepteur p75^{NTR} est également associé au récepteur Nogo et impliqué dans la signalisation des effets inhibiteurs de ces protéines de la myéline vis-à-vis de la croissance axonale. De ce fait, le récepteur p75^{NTR} joue un rôle majeur dans la régulation de la plasticité neuronale et dans les interactions neurones-glie et représente ainsi une cible thérapeutique de choix pour promouvoir la régénération nerveuse.

Au-delà du système nerveux et des maladies neurodégénératives, il a été suggéré que p75^{NTR} pouvait jouer un rôle dans des maladies cardiovasculaires telles que l'athérosclérose et l'ischémie du myocarde (M. L. Bochaton-Pialat et al., Am. J. Pathol., 1995,146, 1-6 ; H. Perlman, Circulation, 1997,95, 981-987). Des travaux récents montrent une augmentation de l'expression de p75^{NTR} et des neurotrophines, et une apoptose massive dans des lésions d'athérosclérose.

Plusieurs études suggèrent également que p75^{NTR} est un médiateur de l'inflammation (Rihl M. et al., Ann. Rheum. Dis. 2005; 64(11):1542-9 ; Raychaudhuri S.P. et al., Prog. Brain. Res. 2004;146: 433-7, Tokuoka S. et al., Br. J. Pharmacol. 2001, 134: 1580-1586).

P75^{NTR} joue également un rôle critique dans la biologie tumorale.

De nombreux composés sont connus pour interagir avec le système trkA/NGF/p75^{NTR} ou pour posséder une activité de type NGF (facteur de croissance neuronale, de l'anglais nerve growth factor). Ainsi la demande de brevet WO 00/59893 décrit des dérivés de pyrimidines substituées qui présentent une activité de type NGF et/ou qui augmentent l'activité du NGF sur les cellules PC12.

La demande de brevet WO03/104225 décrit des composés qui présentent une affinité pour les récepteurs P75^{NTR}. Ces composés sont fortement métabolisés et présentent de forts pourcentages d'inhibition du gène hERG (the human Ether à gogo Related Gene).

Le gène hERG code pour la protéine Kᵥ11.1. d'un canal ionique à potassium. Cette protéine est connue du fait de sa contribution à l'activité électrique du coeur. Quand la capacité du canal à conduire le courant électrique à travers la membrane cellulaire est inhibée par l'action de médicaments, il peut aboutir à un trouble potentiellement fatal appelé le QT syndrome. Un certain nombre de médicaments ont inhibé cette protéine, créant un risque concomitant de mort subite comme un effet secondaire indésirable. Ceci a fait de l'inhibition hERG une question centrale tant dans la régulation des médicaments que dans leur développement (Sanguinetti MC, Tristani-Firouzi M (March 2006). "hERG potassium channels and cardiac arrhythmia". Nature 440 (7083): 463-9).

La présente invention a pour objet des nouveaux composés qui présentent une affinité pour les récepteurs P75^{NTR} et qui ne présentent pas les inconvénients de forte métabolisation et de forte inhibition hERG des composés de l'art antérieur. Elle présente donc un avantage pour le développement de nouveaux médicaments.

La présente invention a pour objet les composés répondant à la formule (I): 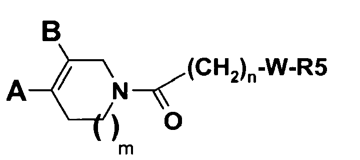 dans laquelle :
- m représente 0 ou 1 ;
- A représente : 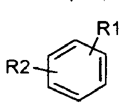
et B représente un atome d'hydrogène
ou
A représente un atome d'hydrogène et B représente : 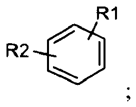
- W- est un hétérocycle azoté choisi parmi : 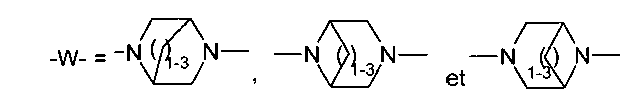
- 1-3 représente 1, 2 ou 3 ;
- n représente 1 ou 2 ;
- R1 représente un atome d'halogène, un groupe (C1-C4)alkyle, un radical trifluorométhyle, un groupe (C1-C4)alkoxy ou un radical trifluorométhoxy ;
- R2 représente un atome d'hydrogène, un atome d'halogène, un groupe (C1-C4)alkyle, un radical trifluorométhyle, un groupe (C1-C4)alkoxy, un radical trifluorométhoxy, un groupe COOR ou un groupe CONH2 ;
- R5 représente un groupe de formule : 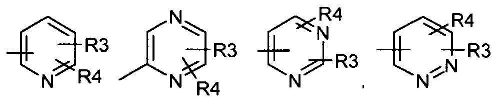 ou 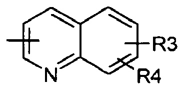
dans lesquels R3 et R4 situés sur l'une quelconque des positions disponibles, représentent indépendamment un atome d'hydrogène, un atome d'halogène, un groupe (C1-C4)alkyle, (C1-C4)alkoxy, un radical trifluorométhyle, trifluorométhoxy un cyano, un groupe COOH, COOalkyle, CONH2, CONR6R7 ou NHCOR ;
- R, R6 et R7 représentent un groupe C1-C6 alkyle.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Dans le cadre de la présente invention, on entend par:
- un atome d'halogène : un fluor, un chlore, un brome ou un iode ;
- un groupe alkyle : un groupe aliphatique saturé linéaire ou ramifié. A titre d'exemples, on peut citer un groupe C1-4 alkyle pouvant représenter un méthyle, éthyle, propyle, isopropyle, butyle, isobutyle, tertbutyle ;
- un groupe fluoroalkyle : un groupe alkyle dont un ou plusieurs atomes d'hydrogène ont été substitués par un atome de fluor ;
- un groupe perfluoroalkyle : un groupe alkyle dont tous les atomes d'hydrogène ont été substitués par un atome de fluor, par exemple trifluoroalkyle ;
- un groupe alcoxy : un radical -O-alkyle où le groupe alkyle est tel que précédemment défini.

Parmi les composés de formule (I) objets de l'invention, un autre groupe de composés est constitué par les composés de formule (I) dans laquelle :
- m représente 0 ou 1 ;
- A représente : 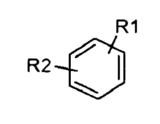 et B représente un atome d'hydrogène
   ou
   A représente un atome d'hydrogène et B représente : 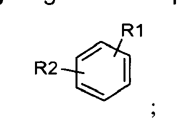
- W est un groupe de formule : 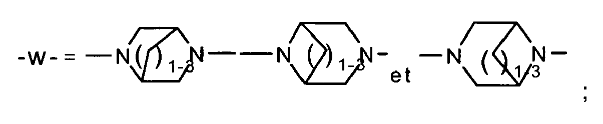 ou bien 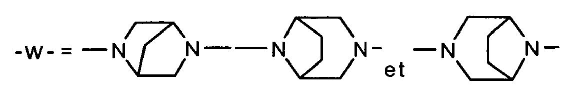
- n = 1 ou 2; ou bien n = 1 ;
- R1 est un atome d'halogène ou un radical trifluorométhyle;
- R2 est un atome d'hydrogène, un radical trifluorométhyle, un groupe COOR ou un groupe CONH2;
- R5 représente un groupe de formule : 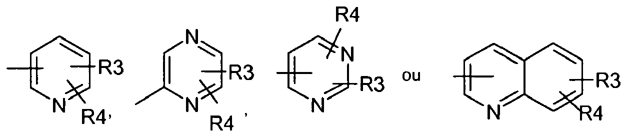
- R3 ou R4 représentent indépendamment un atome d'hydrogène, un halogène, un radical trifluorométhyle, CONH2, COOH ou NHCOCH3;ou bien 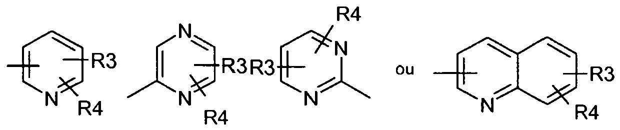
- R3 représentent un atome d'hydrogène, un halogène, un radical trifluorométhyle, CONH2, COOH ou NHCOCH3 et R4 un atome d'hydrogène; à l'état de base ou de sel d'addition à un acide.

Parmi les composés de formule (I) objets de l'invention, un autre groupe de composés est constitué par les composés de formule (I) dans laquelle :
- m représente 1 ;
- A représente : 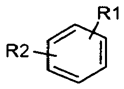 et B représente un atome d'hydrogène ;
- W- est un hétérocycle azoté choisi parmi : 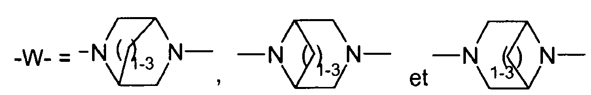
- 1-3 représente 1, 2 ou 3 ;
- n représente 1 ou 2 ;
- R1 représente un atome d'halogène, un groupe (C1-C4)alkyle, un radical trifluorométhyle, un groupe (C1-C4)alkoxy ou un radical trifluorométhoxy ;
- R2 représente un atome d'hydrogène, un atome d'halogène, un groupe (C1-C4)alkyle, un radical trifluorométhyle, un groupe (C1-C4)alkoxy ou un radical trifluorométhoxy ;
- R5 représente un groupe de formule : 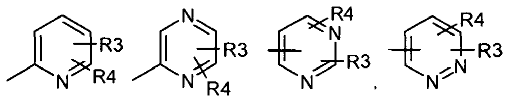 ou 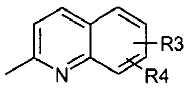
dans lesquels R3 et R4 situés sur l'une quelconque des positions disponibles, représentent indépendamment un atome d'hydrogène, un atome d'halogène, un groupe (C1-C4)alkyle, (C1-C4)alkoxy, un radical trifluorométhyle, trifluorométhoxy, un cyano, un groupe COOH ou COOalkyle.
à l'état de base ou de sel d'addition à un acide.

Parmi les composés de formule (I) objets de l'invention, un autre groupe de composés est constitué par les composés de formule (I) dans laquelle :
- m représente 1 ;
- A représente : 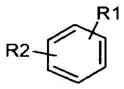 et B représente un atome d'hydrogène ;
- W est un groupe de formule : 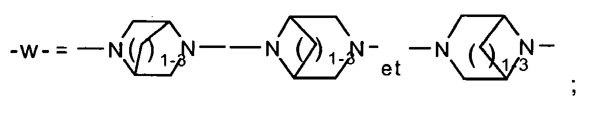 ou bien 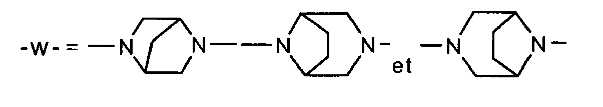
- n = 1 ou 2; ou bien n = 1 ;
- R1 est un atome d'halogène ou un radical trifluorométhyle;
- R2 est un atome d'hydrogène ou radical trifluorométhyle ;
- R5 représente un groupe de formule : 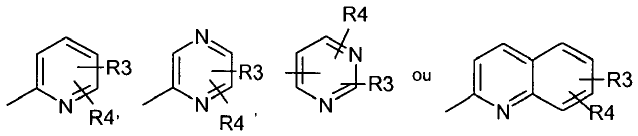
- R3 ou R4 représentent indépendamment un atome d'hydrogène, un halogène ou un radical trifluorométhyle;ou bien 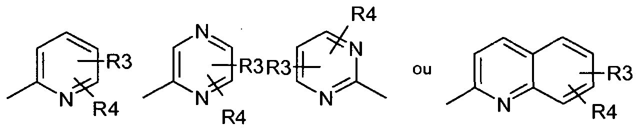
- R3 représente un atome d'hydrogène, un halogène ou un radical trifluorométhyle et R4 un atome d'hydrogène;
à l'état de base ou de sel d'addition à un acide.

Parmi les composés de formule (I) objets de l'invention, on peut notamment citer les composés suivants :
- Composé n°1 : 1-[4-(3-Trifluorométhyl-phényl)-3,6-dihydro-2H-pyridin-1-yl]-2-[8-(5-trifluorométhyl-pyridin-2-yl)-3,8-diaza-bicyclo[3.2.1]oct-3-yl]-éthanone ;
- Composé n°2 : 1-[4-(4-Chloro-phényl)-3,6-dihydro-2H-pyridin-1-yl]-2-(8-pyrimidin-2-yl-3,8-diaza-bicyclo[3.2.1]oct-3-yl)-éthanone ;
- Composé n°3 : 2-(3-Pyrazin-2-yl-3,8-diaza-bicyclo[3.2.1]oct-8-yl)-1-[4-(3-trifluorométhyl-phényl)-3,6-dihydro-2H-pyridin-1-yl]-éthanone ;
- Composé n°4 : 2-(8-Pyrimidin-2-yl-3,8-diaza-bicyclo[3.2.1]oct-3-yl)-1-[4-(3-trifluorométhyl-phényl)-3,6-dihydro-2H-pyridin-1-yl]-éthanone ;
- Composé n°5 : 1-[4-(3-trifluorométhyl-phényl)-3,6-dihydro-2H-pyridin-1-yl]-2-[3-(5-trifluorométhyl-pyridin-2-yl)-3,8-diaza-bicyclo[3.2.1]oct-8-yl]-éthanone ;
- Composé n°6 : 2-(8-Pyridin-2-yl-3,8-diaza-bicyclo[3.2.1]oct-3-yl)-1-[4-(3-trifluorométhyl-phényl)-3,6-dihydro-2H-pyridin-1-yl]-éthanone;
- Composé n°7 : 1-[4-(4-chloro-phényl)-3,6-dihydro-2H-pyridin-1-yl]-2-[8-(5-trifluorométhyl-pyridin-2-yl)-3,8-diaza-bicyclo[3.2.1]oct-3-yl]-éthanone ;
- Composé n°8 : 1-[4-(4-chloro-3-trifluorométhyl-phényl)3,6 dihydro-2H-pyridin-1-yl]-2-[8-(5-trifluorométhyl-pyridin-2-yl)-3,8-diaza-bicyclo[3.2.1]oct-3-yl]-éthanone ;
- Composé n°9: 2-(8-Quinolin-2-yl-3,8-diaza-bicyclo[3.2.1]oct-3-yl)-1-[4-(3-trifluorométhyl-phényl)-3,6-dihydro-2H-pyridin-1-yl]-éthanone;
- Composé n°10 : 1-[4-(4-chloro-phényl)-3,6-dihydro-2H-pyridin-1-yl]-2-[3-(5-trifluorométhyl-pyridin-2-yl)-3,8-diaza-bicyclo[3.2.1]oct-8-yl]-éthanone ;
- Composé n°11 : 1-[4-(4-chloro-3-trifluorométhyl-phényl)-3,6-dihydro-2H-pyridin-1-yl]-2-[8-(5-fluoro-pyrimidin-2-yl)-3,8-diaza-bicyclo[3.2.1]oct-3-yl]-éthanone ;
- Composé n°12 : 2-[8-(5-Bromo-pyrimidin-2-yl)-3,8-diaza-bicyclo[3.2.1]oct-3-yl]-1-[4-(4-chloro-3-trifluorométhyl-phényl)-3,6-dihydro-2H-pyridin-1-yl]-éthanone;
- Composé n°13 : 1-[4-(3-trifluorométhyl-phényl)-3,6 dihydro-2H-pyridin-1-yl]-2-[5-(5-trifluorométhyl-pyridin-2-yl)-2,5-diaza-bicyclo[2.2.1]hept-2-yl]-éthanone :
- Composé n°14:1-[4-(4-Chloro-phényl)-3,6-dihydro-2H-pyridin-1-yl]-2-[5-(5-trifluorométhyl-pyridin-2-yl)-2,5-diaza-bicyclo[2.2.1]hept-2-yl]-éthanone ;
- Composé n°15: 1-[4-(4-Chloro-3-trifluorométhyl-phényl)-3,6-dihydro-2H-pyridin-1-yl]-2-[5-(5-trifluorométhyl-pyridin-2-yl)-2,5-diaza-bicyclo[2.2.1]hept-2-yl]-éthanone ;
- Composé n°16: 1-[4-(4-Chloro-3-trifluorométhyl-phényl)-3,6-dihydro-2H-pyridin-1-yl]-2-(5-pyridin-2-yl-2,5-diaza-bicyclo[2.2.1]hept-2-yl)-éthanone;
- Composé n°17: 1-[4-(4-Chloro-3-trifluorométhyl-phényl)-3,6-dihydro-2H-pyridin-1-yl]-2-[5-(5-fluoro-pyrimidin-2-yl)-2,5-diaza-bicyclo[2.2.1]hept-2-yl]-éthanone;
- Composé n°18: 1-[4-(4-Chloro-3-trifluorométhyl-phényl)-3,6-dihydro-2H-pyridin-1-yl]-2-[9-(5-trifluorométhyl-pyridin-2-yl)-3,9-diaza-bicyclo[3.3.1]non-3-yl]-éthanone;
- Composé n°19: 2-[8-(5-Fluoro-pyrimidin-2-yl)-3,8-diaza-blcyclo[3.2.1]oct-3-yl]-1-[4-(4-trifluorométhyl-phényl)-3,6-dihydro-2H-pyridin-1-yl]-éthanone ;
- Composé n°20: 1-[4-(3-chloro-phényl)-3,6-dihydro-2H-pyridin-1-yl]-2-[8-(5-fluoropyrimidin-2-yl)-3,8-diaza-bicyclo[3.2.1]oct-3-yl]-éthanone ;
- Composé n°21 :2-[8-(5-Fluoro-pyrimidin-2-yl)-3,8-diaza-bicyclo[3.2.1]oct-3-yl]-1-[4-(3-trifluorométhyl-phényl)3,6-dihydro-2H-pyridin-1-yl]-éthanone;
- Composé n°22: 1-[4-(4-chlorol-phényl)-3,6-dihydro-2H-pyridin-1-yl]-2-[8-(5-fluoropyrimidin-2-yl)-3,8-diaza-bicyclo[3.2.1]oct-3-yl]-éthanone :
- Composé n°23: 1-[4-(3,5-Bis-trifluorométhyl-phényl)-3,6-dihydro-2H-pyridin-1-yl]-2-[8-(5-fluoro-pyrimidin-2-yl)-3,8-diaza-bicyclo[3.2.1]oct-3-yl]-éthanone;
- Composé n°24: 2-[8-(5-Fluoro-pyrimidin-2-yl)-3,8-diaza-bicyclo[3.2.1]oct-3-yl]-1-(4-m-tolyl-3,6-dihydro-2H-pyridin-1-yl)-éthanone;
- Composé n°25: 1-[4-(4-chloro-3-trifluorométhyl-phényl)-3,6-dihydro-2H-pyridin-1-yl]-2-[8-(pyrimidin-2-yl)-3,8-diaza-bicyclo[3.2.1]oct-3-yl]-éthanone ;
- Composé n°26: 1-[4-(4-Chloro-3-trifluorométhyl-phényl)-3,6-dihydro-2H-pyridin-1-yl]-2-[3-(5-fluoro-pyrimidin-2-yl)-3,8-diaza-bicyclo[3.2.1]oct-8-yl]-éthanone ;
- Composé n°27: 1-[4-(4-Chloro-3-trifluorométhyl-phényl)-3,6-dihydro-2H-pyridin-1-yl]-2-(8-pyridin-3-yl-3,8-diaza-bicyclo[3.2.1]oct-3-yl)-éthanone;
- Composé n°28: Méthyl ester de l'acide 6-(3-{2-[4-(4-Chloro-3-trifluorométhylphényl)-3,6-dihydro-2H-pyridin-1-yl]-2-oxo-éthyl}-3,8-diaza-bicyclo[3.2.1]oct-8-yl)-nicotinique ;
- Composé n°29 : Acide 6-(3-{2-[4-(4-Chloro-3-trifluorométhyl-phényl)-3,6-dihydro-2H-pyridin-1-yl]-2-oxo-éthyl}-3,8-diaza-bicyclo[3.2.1]oct-8-yl)-nicotinique ;
- Composé n°30: 1-[4-(4-Chloro-3-trifluorométhyl-phényl)-3,6-dihydro-2H-pyridin-1-yl]-2-[8-(6-trifluorométhyl-pyridin-3-yl)-3,8-diaza-bicyclo[3.2.1]oct-3-yl]-éthanone ;
- Composé n°31: 2-[8-(5-Chloro-pyridin-2-yl)-3,8-diaza-bicyclo[3.2.1]oct-3-yl]-1-[4-(4-chloro-3-trifluorométhyl-phényl)-3,6-dihydro-2H-pyridin-1-yl]-éthanone ;
- Composé n°32: 1-[4-(4-Chloro-3-trifluorométhyl-phényl)-3,6-dihydro-2H-pyridin-1-y)]-2-(8-quinolin-2-yl-3,8-diaza-bicyclo[3.2.1]oct-3-yl)-éthanone ;
- Composé n°33: 1-[4-(4-Chloro-3-trifluorométhyl-phényl)-3,6-dihydro-2H-pyridin-1-yl]-2-[8-(5-fluoro-pyridin-2-yl)-3,8-diaza-bicyclo[3.2.1]oct-3-yl]-éthanone ;
- Composé n°34: 2-[8-(6-Chloro-pyridin-3-yl)-3,8-diaza-bicyclo[3.2.1]oct-3-yl]-1-[4-(4-chloro-3-trifluorométhyl-phényl)-3,6-dihydro-2H-pyridin-1-yl]-éthanone ;
- Composé n°35: 1-[4-(3-Trifluorométhyl-phényl)-3,6-dihydro-2H-pyridin-1-yl]-2-[8-(3-trifluorométhyl-pyridin-2-yl)-3,8-diaza-bicyclo[3.2.1]oct-3-yl]-éthanone ;
- Composé n°36: éthyl ester de l'acide 6-(3-{2-[4-(4-Chloro-3-trifluorométhylphényl)-3,6-dihydro-2H-pyridin-1-yl]-2-oxo-éthyl}-3,8-diaza-bicyclo[3.2.1]oct-8-yl)-nicotinique ;
- Composé n°37: 2-(8-Pyrazin-2-yl-3,8-diaza-bicyclo[3.2.1]oct-3-yl)-1-[4-(3-trifluorométhyl-phényl)-3,6-dihydro-2H-pyridin-1-yl]-éthanone ;
- Composé n°38: 2-(8-Pyrimidin-4-yl-3,8-diaza-bicyclo[3.2.1]oct-3-yl)-1-[4-(3-trifluorométhyl-phényl)-3,6-dihydro-2H-pyridin-1-yl]-éthanone ;
- Composé n°39: 1-[4-(4-Chloro-3-trifluorométhyl-phényl)-3,6-dihydro-2H-pyridin-1-yl]-2-(8-pyrazin-2-yl-3,8-diaza-bicyclo[3.2.1]oct-3-yl)-éthanone ;
- Composé n°40: 2-(8-Pyrazin-2-yl-3,8-diaza-bicyclo[3.2.1]oct-3-yl-1-(4-m-tolyl-3,6-dihydro-2H-pyridin-1-yl)-éthanone ;
- Composé n°41: méthyl ester de l'acide 2-(3-{2-[4-(4-Chloro-3-trifluorométhylphényl)-3,6-dihydro-2H-pyridin-1-yl]-2-oxo-éthyl}-3,8-diaza-bicyclo[3.2.1]oct-8-yl)-pyrimidine-5-carboxylique ;
- Composé n°42: acide 2-(3-{2-[4-(4-Chloro-3-trifluorométhyl-phényl)-3,6-dihydro-2H-pyridin-1-yl]-2-oxo-éthyl}-3,8-diaza-bicyclo[3.2.1]-oct-8-yl)-pyrimidine-5-carboxylique ;
- Composé n°43: 1-[4-(4-Chloro-3-trifluorométhyl-phényl)-3,6-dihydro-2H-pyridin-1-yl]-3-[8-(5-fluoro-pyrimidin-2-yl)-3,8-diaza-bicyclo[3.2.1]oct-3-yl]-propan-1-one ;
- Composé n°44: 1-[4-(4-Chloro-3-trifluorométhyl-phényl)-3,6-dihydro-2H-pyridin-1-yl]-2-[5-(5-fluoro-pyrimidin-2-yl)-2,5-diaza-bicyclo[2.2.2]oct-2-yl]-éthanone ;
- Composé n°45: 2-[8-(5-Fluoro-pyrimidin-2-yl)-3,8-diaza-bicyclo[3.2.1]oct-3-yl]-1-[4-(3-méthoxy-phényl)-3,6-dihydro-2H-pyridin-1-yl]-éthanone ;
- Composé n°46: 1-[4-(4-Chloro-3-trifluorométhyl-phényl)-3,6-dihydro-2H-pyridin-1-yl]-2-[5-(6-trifluorométhyl-pyridazin-3-yl)-2,5-diaza-bicyclo[2.2.1]hept-2-yl]-éthanone ;
- Composé n°47: 2-[8-(5-Fluoro-pyrimidin-2-yl)-3,8-diaza-bicyclo[3.2.1]oct-3-yl]-1-[3-(3-trifluorométhyl-4-chloro-phényl)-2,5-dihydro-pyrrol-1-yl]-éthanone;
- Composé n°48: 6-(3-{2-[4-(4-Chloro-3-trifluorométhyl-phényl)-3,6-dihydro-2H-pyridin-1-yl]-2-oxo-éthyl}-3,8-diaza-bicyclo[3.2.1]oct-8-yl)-nicotinamide ;
- Composé n°49: 2-[8-(5-Fluoro-pyrimidin-2-yl)-3,8-diaza-bicyclo[3.2.1]oct-3-yl]-1-[4-(2,3-dichloro-phényl)-3,6-dihydro-2H-pyridin-1-yl]-éthanone;
- Composé n°50: 1-[4-(4-Chloro-3-trifluorométhyl-phényl)-3,6-dihydro-2H-pyridin-1-yl]-2-[8-(6-fluoro-pyridin-3-yl)-3,8-diaza-bicyclo[3.2.1]oct-3-yl]-éthanone ;
- Composé n°51: 2-[8-(5-Fluoro-pyrimidin-2-yl)-3,8-diaza-bicyclo[3.2.1]oct-3-yl]-1-[5-(3-trifluorométhyl-phényl)-3,6-dihydro-2H-pyridin-1-yl]-éthanone ;
- Composé n°52: 2-[8-(5-Fluoro-pyrimidin-2-yl)-3,8-diaza-bicyclo[3.2.1]oct-3-yl]-1-[3-(3-trifluorométhyl-phényl)-2,5-dihydro-pyrrol-1-yl]-éthanone ;
- Composé n°53: Méthyl ester de l'acide 3-(1-{2-[8-(5-Fluoro-pyrimidin-2-yl)-3,8-diaza-bicyclo[3.2.1]oct-3-yl]-acétyl}-1,2,5,6-tétrahydro-pyridin-3-yl)-benzoique ;
- Composé n°54: 2-[8-(5-Fluoro-pyrimidin-2-yl)-3,8-diaza-bicyclo[3.2.1]oct-3-yl]-1-[5-(2-trifluorométhyl-phényl)-3,6-dihydro-2H-pyridin-1-yl]-éthanone ;
- Composé n°55: 1-[4-(4-Chloro-3-trifluorométhyl-phényl)-3,6-dihydro-2H-pyridin-1-yl]-2-(8-pyrimidin-5-yl-3,8-diaza-bicyclo[3.2.1]oct-3-yl)-éthanone.
- Composé n°56: 2-[8-(5-Fluoro-pyrimidin-2-yl)-3,8-diaza-bicyclo[3.2.1]oct-3-yl]-1-[4-(3-trifluorométhoxyl-phényl)-3,6-dihydro-2H-pyridin-1-yl]-éthanone;
- Composé n°57: 1-[4-(4-Chloro-3-trifluorométhyl-phényl)-3,6-dihydro-2H-pyridin-1-yl]-2-[5-(6-trifluorométhyl-pyridazin-3-yl)-2,5-diaza-bicyclo[2.2.2]oct-2-yl]-éthanone ;
- Composé n°58: N-[6-(3-{2-[4-(4-Chloro-3-trifluorométhyl-phényl)-3,6-dihydro-2H-pyridin-1-yl]-2-oxo-éthyl}-3,8-diaza-bicyclo[3.2.1]oct-8-yl)-pyridin-3-yl]-acétamide ;
- Composé n°59: 2-(8-Quinolin-3-yl-3,8-diaza-bicyclo[3.2.1]oct-3-yl)-1-[4-(3-trifluorométhy-14-chloro -phényl)-3,6-dihydro-2H-pyridin-1-yl]-éthanone;
à l'état de base ou de sel d'addition à un acide.

Dans ce qui suit, on entend par "groupe protecteur Pg" un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans Protective Groups in Organic Synthesis, Green et al., 2nd Edition (John Wiley & Sons, Inc., New York).

Conformément à l'invention, on peut préparer les composés de formule générale (I) selon le procédé qui suit.

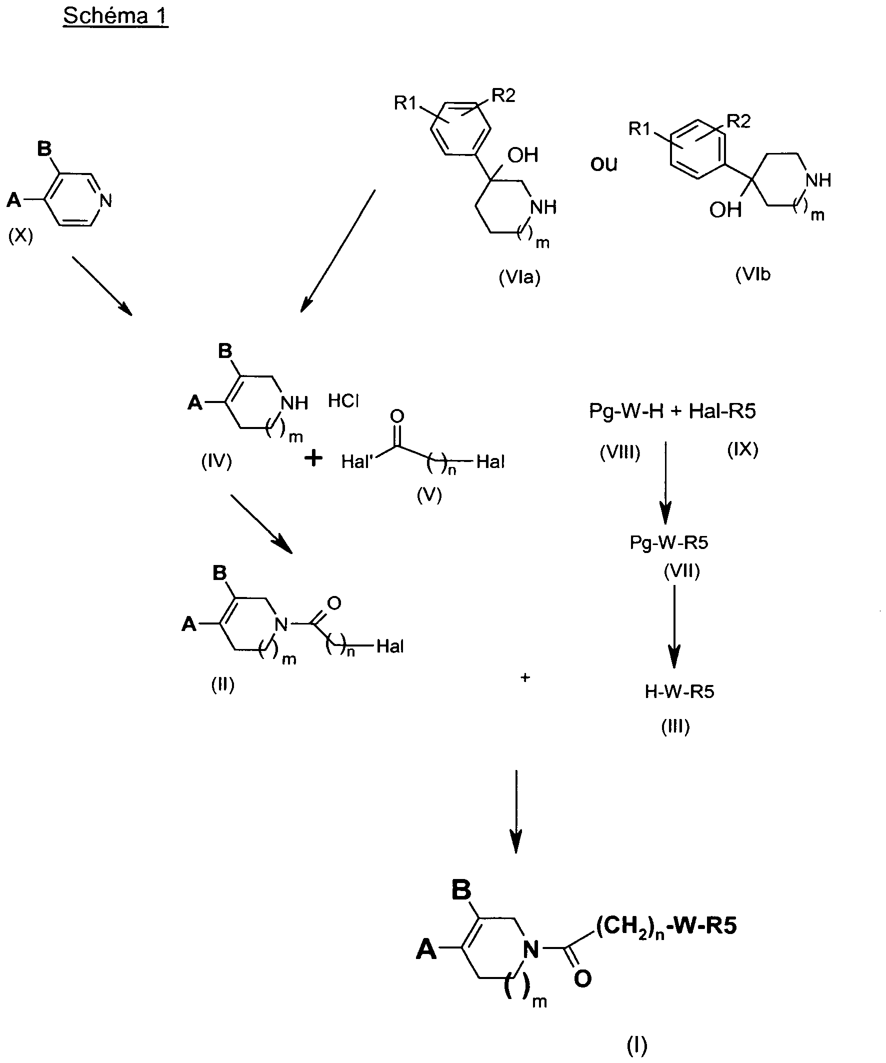

Plus précisément, le procédé de préparation des composés de formule générale (I) dans laquelle A, B, m, n, W, R5 sont tels que définis précédemment comprend la réaction d'un composé de formule (II) : 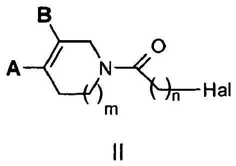 dans laquelle A, B, m, n sont définis comme en formule générale (I) et Hal représente un atome d'halogène, par exemple le chlore, et d'un composé de formule générale (III) :

H-W-R5 (III)

dans laquelle W et R5 sont définis comme en formule générale (I), selon des méthodes connues de l'homme du métier, par exemple en présence d'une base, dans un solvant tel que décrit dans WO 03/104225. Ainsi, à titre de base, on peut citer les bases organiques telles que la triéthylamine, la N,N-diisopropylamine, la Diisopropyléthylamine (DPEA) ou la N-méthyl-morpholine ou les carbonates ou bicarbonates de métal alcalin tel que le carbonate de potassium, le carbonate de sodium ou le bicarbonate de sodium et en l'absence ou en présence d'un iodure de métal alcalin tel que l'iodure de potassium ou l'iodure de sodium. La réaction s'effectue dans un solvant tel que l'acétonitrile, le N,N-diméthylformamide (DMF), I'N-méthyl-pyrrodinone le toluène ou le propan-2-ol et à une température comprise entre la température ambiante et la température de reflux du solvant. Par température ambiante, on entend une température comprise entre 5 et 25°C. A titre d'exemple la réaction peut s'effectuer en présence de bicarbonate de sodium, d'iodure de sodium dans un solvant tel que le DMF.

Dans les produits de formule générale (I) ainsi obtenus, R, R1, R2, R3 R4, R6, et R7 peuvent être modifiés par des traitements communément utilisés par l'homme du métier, comme par exemple par hydrolyse d'un groupe ester pour donner un groupe carboxylique.

Généralement, les sels d'addition acide des composés de formule générale (I) peuvent être obtenus par addition de l'acide approprié, tel que l'acide chlorhydrique, l'acide bromhydrique, l'acide oxalique.

Les composés de formule (III), éventuellement sous forme de sels peuvent être préparés à partir des composés correspondants de formule (VII) :

Pg-W-R5 (VII)

dans laquelle W et R5 sont tels que définis en formule (I) et Pg représente un groupe protecteur d'un atome d'azote de W. De préférence, Pg est un groupe benzyle et la déprotection s'effectue selon des méthodes classiques, bien connues de l'homme du métier, par exemple par hydrogénation catalytique sur Pd/C ou par traitement avec des chloroformiates puis hydrolyse en milieu acide.

Les composés de formule (VII) peuvent être préparés à partir des composés de formule (VIII) :

Pg-W-H (VIII)

et (IX) :

Hal-R5 (IX)

dans lesquelles Pg, W, R5 sont définis comme précédemment et Hal représente un atome d'halogène, de préférence le chlore. Cette réaction s'effectue généralement dans les mêmes conditions que la réaction de préparation des composés de formule (I) à partir des composés de formule (II) et (III).

Alternativement, les composés de formule (III) peuvent être préparés par la méthode de couplage de Buchwald en présence d'un catalysateur au Palladium et d'une phosphine opportunément choisis, en utilisant comme solvant des solvants inertes tels que le toluène ou le xylène, à une température comprise entre la température ambiante et 110°C.

Des exemples des telles réactions sont décrits dans la partie expérimentale.

Les composés de formule (II) peuvent être obtenus par réaction d'un composé correspondant de formule (IV) :

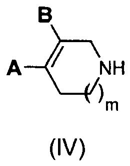

Dans laquelle A, B, m sont définis comme en formule générale (I) ; éventuellement sous forme de sel d'addition acide, et d'un composé de formule (V) : 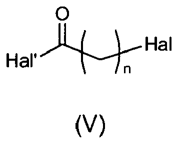 dans laquelle Hal et n sont tels que définis en formule (II) et Hal' représente un atome d'halogène, identique ou différent de Hal. De préférence, Hal' représente un atome de chlore.

Cette réaction s'effectue généralement en présence d'une base, telle que la triéthylamine, la N,N-diisopropyléthylamine ou la N-méthylmorpholine, dans un solvant tel que le dichlorométhane, le chloroforme, le tétrahydrofuranne, le dioxane ou un mélange de ces solvants et à une température comprise entre 0°C et la température ambiante. Les composés de formule (V) sont généralement disponibles commercialement.

Les composés de formule (IV) éventuellement sous forme de sel d'addition acide, peuvent être obtenus à partir des composés de formule (VI) (a) ou (b): 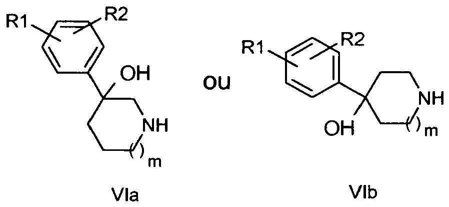 dans lesquelles R1, R2 et m sont tels que définis précédemment, par déshydratation.

La déshydratation s'effectue en milieu acide, en utilisant par exemple l'acide chlorhydrique concentré ou un mélange acide acétique-acide chlorhydrique ou un mélange acide acétique-acide sulfurique, à une température comprise entre la température ambiante et 140°C. On peut également effectuer la réaction en utilisant l'acide p-toluène sulfonique dans un solvant tel que le toluène et à une température comprise entre la température ambiante et la température de reflux.

Alternativement, les composés de formule (IV), quand m est = 1, peuvent être préparés à partir d'un composé de formule (X) 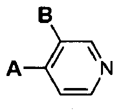 dans laquelle A et B sont tels que définis précédemment, par formation du sel de benzyl ammoniun quaternaire, suivi par une réduction avec du sodium borohydrure dans des solvants comme le méthanol ou le dioxane à une température comprise entre O°C et la température ambiante, et par une réaction de débenzylation effectuée selon des méthodes classiques, bien connues de l'homme du métier, par exemple par hydrogénation catalytique sur Pd/C ou par traitement avec des chloroformiates puis hydrolyse en milieu acide.

Des exemples des telles réactions sont décrits dans la partie expérimentale.

Eventuellement, le procédé selon l'invention comprend l'étape ultérieure consistant à isoler le produit désiré obtenu.

Les produits de formules (VI), (V), (VIII), (IX), (X) et les réactifs, quand leur mode de préparation n'est pas décrit, sont disponibles dans le commerce ou décrits dans la littérature, ou bien peuvent être préparés selon des méthodes qui sont décrites ou connues de l'homme du métier.

Selon un autre de ses aspects, l'invention a également pour objet des composés de formule (II) 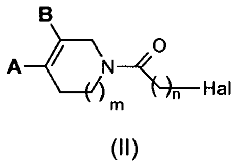 dans laquelle A, B, m, n et Hal sont définis comme précédemment ; à l'état de base ou de sel d'addition à un acide. Ces composés sont utiles comme intermédiaires de synthèse des composés de formule (I).

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

Les mesures physico-chimiques ont été effectuées de la façon suivante :

Les points de fusion ont été mesurés avec un appareil Buchi B540.

Les spectres de résonnance magnétique nucléaire du proton (RMN 1H) ont été enregistrés dans les conditions suivantes:
a) à 500 MHz sur un appareil Bruker équipé avec une console Avance III ;
b) à 400MHz sur un appareil Bruker équipé avec une console Avance I.

Les déplacements chimiques sont rapportés en ppm par rapport à la fréquence TMS.

Touts les spectres ont étés enregistrés à la température de 40°C.

Les abréviations utilisées pour caractériser les signaux sont les suivantes: s = singulet, bs =singulet large, m = multiplet, bm= multiplet large, d = doublet, bd= doublet large, t = triplet, q = quadruplet.
* = non intégrable à cause de l'interférence avec un pic large du à l'eau.
** = non intégrable à cause de l'interférence avec un pic du au solvant RMN. 2Xm= deux multiplets partiellement superposés.

L'HPLC a été effectuée au moyen d'un système ThermoElectron LCQ Deca XP Max équipé avec un détecteur à spectrométrie de masse à trappe d'ions ainsi qu'un détecteur à barre de diodes.

Les conditions d'analyse par chromatographie liquide couplée à la spectrométrie de masse (LC/UV/MS) sont les suivantes:
- système chromatographique A
- Eluant A = H2O + 0,01 % TFA
- Eluant B = CH3CN
- Gradient de 98% de A à 95% de B en 10 minutes, puis élution par 95% de B pendant 5 minutes.
- Débit 0,5 ml/minute; température 40°C
- Injection de 2 µL de solution à 0,1mg/ml dans un mélange CH3CN : H2O = 9 :1
- système chromatographique B
- Eluant A = H2O + 0,05% TFA
- Eluant B = CH3CN + 0,035% TFA
- Gradient de 98% de A à 95% de B en 12 minutes, puis élution par 95% de B pendant 3 minutes.
- Débit 0,7 ml/minute; température 40°C
- Injection de 2 µL de solution à 0,1mg/ml dans un mélange CH3CN : H2O = 9 :1
- système chromatographique C
- Eluant A = Tampon acétate d'ammonium 5mM pH 6,5
- Eluant B = CH3CN
- Gradient de 98% de A à 95% de B en 10 minutes, puis élution par 95% de B pendant 5 minutes.
- Débit 0,5 ml/minute; température 40°C
- Injection de 2 µL de solution à 0,1mg/ml dans un mélange CH3CN : H2O = 9 :1
- système chromatographique D
- Eluant A = H2O + 0,005% TFA
- Eluant B = CH3CN
- Gradient de 95% de A à 90% de B en 17 minutes, puis élution par 90% de B pendant 5 minutes.
- Débit 0,3 ml/minute; température 30°C
- Injection de 2 µL de solution à 0,1mg/ml dans un mélange CH3CN : H2O = 9 :1
- système chromatographique E
- Eluant A = Tampon acétate d'ammonium 5mM pH 6,5
- Eluant B = CH3CN
- Gradient de 95% de A à 90% de B en 17 minutes, puis élution par 90% de B pendant 5 minutes.
- Débit 0,3 ml/minute; température 30°C
- Injection de 2 µL de solution à 0,1mg/ml dans un mélange CH3CN : H2O = 9 :1
- système chromatographique F
- Eluant A = H2O + 0,005% TFA
- Eluant B = CH3CN
- Gradient de 95% de A à 90% de B en 22 minutes, puis élution par 90% de B pendant 7 minutes.
- Débit 0,3 ml/minute; température 40°C
- Injection de 2 µL de solution à 0,1mg/ml dans un mélange CH3CN : H2O = 9 :1
- système chromatographique G
- Eluant A = H2O + 0,01% TFA
- Eluant B = CH3CN
- Gradient de 80% de A à 60% de B en 15 minutes, puis de 60% de A à 100% de B en 5 minutes, puis élution par 100% de B pendant 5 minutes.
- Débit 0,4 ml/minute; température 40°C
- Injection de 2 µL de solution à 0,1mg/ml dans un mélange CH3CN : H2O = 9 :1
- système chromatographique H
- Eluant A = H2O + 0,01% TFA
- Eluant B = CH3CN
- Gradient de 80% de A à 95% de B en 19 minutes, puis élution par 95% de B pendant 3 minutes.
- Débit 0,5 ml/minute; température 40°C
- Injection de 2 µL de solution à 0,1mg/ml dans un mélange CH3CN : H2O = 9 :1

Les produits sont détectés en UV à 220 nm.

Les colonnes utilisées sont des C18 avec une granulometrie entre 2 et 4 µm en preference de 3,5 µm.

Pour la partie spectrométrie de masse :
- Mode d'ionisation: électrospray positif (ESI+)
- Balayage de 100 à 1200 uma

La chromatographie en couche mince a été effectuée sur des plaques CCM de gel de silice Merck Silica gel 60. Le gel de silice pour la chromatographie sur colonne flash est commercialisé par Biotage.

Tous les solvants utilisés sont de pureté "reagent grade" ou "HPLC grade".

### Préparation 1

### Chlorhydrate de 2-[8-(5-trifluorométhyl-pyridin-2-yl)-3,8-diazabicyclo[3.2.1]octane

On charge 0,9 g de 2-chloro-5(trifluorométhyl)pyridine (IX), 1g de 1-benzyl-3,8-diaza-bicyclo[3.2.1]octane , 0,75 g de carbonate de potassium et 0,33 g de Nal dans 8 mL de DMF. La réaction est conduite dans un initiateur à micro-ondes Biotage® pendant 30 mn à 160°C. Puis on verse dans une solution aqueuse saturée en chlorure de sodium et on extrait avec de l'acétate d'éthyle. La phase organique est séchée sur Na₂SO₄, filtrée et évaporée sous vide. On isole 1,5 g d'une matière huileuse que l'on purifie par flash chromatographie sur colonne Biotage®, au moyen de l'éluant cyclohexane 98/acétate d'éthyle 2. On isole 440 mg d'une huile claire. On hydrogène à 45°C sous pression atmosphérique pendant 4 heures 0,44 g du composé obtenu à l'étape précédente dans 20 mL d'éthanol, 2 mL d'isopropanol.HCl, en présence de 0,14 g de Pd/C à 10%. On filtre, on évapore sous vide et on isole 350 mg du produit du titre, sous forme de solide blanc.

### Préparation 2

### Chlorhydrate de 2-[8-(5-fluoro-pyrimidin-2-yl)-3,8-diaza-bicyclo[3.2.1]octane

On charge 1,44 g de 2-chloro-5-fluoropyrimidine, 2,2g de 1-benzyl-3,8-diazabicyclo[3.2.1]octane , 1,7 g de carbonate de potassium et 0,73 g de Nal dans 27 mL de N-méthylpyrrolidone. On chauffe à 110°C pendant 5 heures. Puis on verse dans une solution aqueuse saturée en chlorure de sodium et on extrait avec de l'acétate d'éthyle. La phase organique est séchée sur Na₂SO₄, filtrée et évaporée sous vide. On isole 3,2 g d'une matière huileuse que l'on purifie par flash chromatographie sur colonne Biotage®, au moyen de l'éluant cyclohexane 95/acétate d'éthyle 5. On isole 1,4 g de solide blanc que l'on dissous dans 35 ml de 1,2-dichloroéthane. On ajoute à 0°C 0,72 ml de 1-chloroéthylchloroformate et laisse agiter sous flux d'azote pendant 10 minutes à 0°C et ensuite 3 heures à 85°C. On évapore le solvant et on ajoute 35 ml de méthanol. On chauffe pendant 30 minutes à la température de reflux. On évapore le solvant et on traite le résidu avec de l'isopropanol. On obtient un solide blanc que l'on filtre et on isole 900 mg de produit du titre. Pf 236-239°C

### Préparation 3

### Chlorhydrate de 2-[8-(3-pyridin-2-yl)-3,8-diaza-bicyclo[3.2.1]octane

On charge 0,39 g de 3-bromo-pyridine, 1g de 1-benzyl-3,8-diazabicyclo[3.2.1]octane, 0,07 g de palladium acétate, 0,34 g de sodium t-butoxyde et 0,06 g de tri-t-butyl phosphine dans 8 mL de o-xilène. On chauffe à 120°C pendant 6 heures. On filtre sur célyte et on évapore le solvant. On isole 1,3 g d'une matière huileuse que l'on purifie par flash chromatographie sur colonne Biotage®, au moyen de l'éluant cyclohexane 6/acétate d'éthyle 4. On isole 700 mg d'une huile claire. On hydrogène à 40°C sous pression atmosphérique pendant 4 heures le produit de l'étape précédente, dans 29 mL d'éthanol, 2 mL d'isopropanol.HCl, en présence de 0,35 g de Pd/C à 10%. On filtre, on évapore sous vide et on isole 500 mg du produit du titre, sous forme de solide blanc.

### Préparation 4

### Chlorhydrate du méthyl ester de l'acide 3,8-diaza-bicyclo[3.2.1]oct-8-yl-nicotinique

On charge 0,42 g de 6-chloronicotinate de méthyle (IX), 0,5g de 1-benzyl-3,8-diaza-bicyclo[3.2.1]octane , 0,4 g de carbonate de potassium et 0,17 g de Nal dans 7 mL de N-méthylpyrrolidone. On chauffe pendant 7 heures à 110°C. Puis on verse dans une solution aqueuse saturée en chlorure de sodium et on extrait avec de l'acétate d'éthyle. La phase organique est séchée sur Na₂SO₄, filtrée et évaporée sous vide. On isole 1,1 g d'une matière huileuse que l'on purifie par flash chromatographie sur colonne Biotage®, au moyen de l'éluant cyclohexane 8/acétate d'éthyle 2. On isole 520 mg d'une huile claire. On hydrogène à 40°C sous pression atmosphérique pendant 2 heures le produit obtenu à l'étape précédente, dans 20 mL d'éthanol, 2 mL d'isopropanol.HCl, en présence de 0,22 g de Pd/C à 10%. On filtre, on évapore sous vide et on isole 440 mg du produit du titre, sous forme de solide blanc.

### Préparation 5

### Chlorhydrate de 4-(3-trifluorométhyl-phényl)-1,2,3,6,-tétrahydropyridine

Dans un ballon muni d'un agitateur mécanique, on charge 50 g de 4-(3-trifluorométhyl-phényl)-4-piperidinol dans 377 mL d'eau et 514 mL d'acide chlorhydrique concentré. On laisse réagir à reflux pendant 5 heures puis on refroidit à température ambiante. Un solide blanc précipite. On le filtre sur Büchner et on le sèche à l'étuve. On isole 37 g d'un solide blanc. Pf 203-204°C

### Préparation 6

### Chlorhydrate de 4-(3-trifluorométhyl-4-chloro-phényl)-1,2,3,6,-tétrahydropyridine.

En opérant comme décrit dans la préparation 5, mais en utilisant le 4-(3-trifluorométhyl-4-chloro-phényl)-4-piperidinol au lieu du 4-(3-Trifluorométhyl-phényl)-4-piperidinol on obtient le composé du titre. Pf 263-265°C

### Préparation 7

### Chlorhydrate du méthyl ester de l'acide 3-(1,2,5,6-tétrahydro-pyridin-3-yl)-benzoique

On charge 10,9 g de l'acide 3-(pyridin-3-yl)-benzoique méthyl ester, 90 ml de toluène, 8,72 g de bromure de benzyle, et on chauffe à la température de reflux pendant 8 heures. On évapore et traite avec du diisopropyl éther. On obtient 19,65 g d'un solide que l'on dissous dans 540 ml de méthanol. On ajoute lentement à 0°C 3,7 g de sodium borohydrure et on agite à température ambiante pendant 30 minutes.

On évapore le solvant, on reprend le résidu avec de l'eau et on extrait avec de l'acétate d'éthyle. La phase organique est séchée sur Na₂SO₄, filtrée et évaporée sous vide. On isole 18 g d'une matière huileuse que l'on purifie par flash chromatographie au moyen de l'éluant cyclohexane 8/acétate d'éthyle 2. On isole 10 g d'une huile. On hydrogène à 40°C sous pression atmosphérique pendant 2 heures de produit de l'étape précédente, dans 200 mL de méthanol, 5 mL d'isopropanol.HCl, en présence de 1,2 g de Pd/C à 10%. On filtre, on évapore sous vide et on isole 440 mg du produit du titre, sous forme de solide blanc. P.f. 160-162°C

### Préparation 8

### 2-Chloro-1-[4-(3-trifluorométhyl-phényl)-3,6-dihydro-2H-pyridin-1-yl]-éthanone

Dans un ballon muni d'un agitateur magnétique, on met en suspension 3,44 g du composé de la préparation 5 dans 33,5 mL de dichlorométhane. On ajoute 3,8 mL de triéthylamine et on porte à 0°C. A 0°C, on coule goutte à goutte 1,01 mL de chloroacétylchlorure, c'est-à-dire le composé de formule générale (V) dans laquelle Hal=Hal'=Cl et n=1. On laisse réagir pendant 1 heure et demie et on verse dans de l'eau. On extrait au dichlorométhane. La phase organique est séchée sur Na₂SO₄, filtrée et évaporée sous vide. On isole 4,1 g d'une graisse huileuse et sombre que l'on triture puis que l'on laisse reposer au froid. On décante et on évapore le surnageant sous vide. On isole 420 mg d'une huile claire.

### Préparation 9

### 2-Chloro-1-[4-(3-trifluorométhyl-4-chloro-phényl)-3,6-dihydro-2H-pyridin-1-yl]-éthanone

En opérant comme décrit dans la préparation 8, mais en utilisant le composé de la préparation 6 au lieu du composé de la préparation 5 et en purifiant le brut par flash chromatographie, on obtient le composé du titre sous forme de solide blanc.

### Préparation 10

### Méthyl ester de l'acide 2-(Chloro-acétyl)-1,2,5,6-tétrahydro-pyridin-3-yl-benzoique

En opérant comme décrit dans la préparation 9, mais en utilisant le composé de la préparation 7 au lieu du composé de la préparation 6 on obtient le composé du titre sous forme de solide blanc.

### EXEMPLE 1

### Composé n°1 :1-[4-(3-trifluorométhyl-phényl)-3,6-dihydro-2H-pyridin-1-yl]-2-[8-(5-trifluorométhyl-pyridin-2-yl)-3,8-diaza-bicyclo[3.2.1]oct-3-yl]-éthanone et son oxalate

On fait réagir 0,19 g du composé obtenu à la préparation 1, 0,18 g du composé obtenu à la préparation 8, 0,18 g de carbonate de potassium et 0,04 g de Nal dans 4,5 mL de DMF. La réaction est conduite au moyen d'un initiateur Biotage® à micro-ondes pendant 30 mn à 180°C. On verse dans l'eau et on extrait à l'acétate d'éthyle. La phase organique est séchée sur Na₂SO₄, filtrée et évaporée sous vide. On isole 250 mg d'une matière huileuse. On purifie sur colonne par flash chromatographie au moyen d'une colonne Biotage® que l'on élue avec un mélange cyclohexane 8/acétate d'éthyle 2. On isole 100 mg d'huile. Par salification avec l'acide oxalique, on obtient 80 mg d'un solide blanc.

RMN (Appareil b). δ (ppm, dmso-d6): 1,80 - 2,05 (m, 4H), 2,41 - 2.,6 (m, **), 2,59 - 2,84 (m, 3H), 3.,5 (bs, 1H), 3,31 (bs, 1H), 3,69 (m, 1H), 3,76 (m, 1H), 4,12 (s, 1 H), 4,33 (s, 1 H), 4,66 (bs, 2H), 6,36 (m, 1 H), 6,87 (m, 1 H), 7,57 - 7,67 (m, 2H), 7,70 - 7,82 (m, 3H), 8,40 (bs, 1 H).

### EXEMPLE 2

### Composé n°11: 1-[4-(3-trifluorométhyl-4-chloro-phényl)-3,6-dihydro-2H-pyridin-1-yl]-2-[8-(5-fluoro-pyrimidin-2-yl)-3,8-diaza-bicyclo[3.2.1]oct-3-yl]-éthanone

On met à réagir 40,1 g du composé obtenu à la préparation 9, 26,6 g du composé obtenu à la préparation 2, 30 ml diisopropyl-éthyl-amine et 1500 mL de DMF. On chauffe pendant 3 heures à 100°C. On verse dans l'eau et on extrait à l'acétate d'éthyle. La phase organique est séchée sur Na₂SO₄, filtrée et évaporée sous vide. On isole 69 g d'une matière huileuse. On purifie sur colonne par flash chromatographie au moyen d'une colonne que l'on élue avec un mélange hexane 7/acétate d'éthyle 3. On isole 42,14 g du produit du titre. On traite avec du diéthyl éther, on filtre et on obtient un solide blanc de 33,45 g.

RMN (Appareil a). δ (ppm, dmso-d6): 1,72 - 1,98 (m, 4H), 2,38 (m, 2H), 2,45 - 2,54 (m, **), 2,56 - 2,74 (m, 3H), 3,14 (s, 1H), 3,18 (s, 1H), 3,68 (m, 1H), 3,78 (m, 1 H), 4,12 (bs, 1 H), 4,36 (bs, 1 H), 4,59 (m, 2H), 6,38 + 6,41 (2Xm, 1 H), 7,70 (d, J= 8,5Hz, 1 H), 7,75 (bd, J= 8,5Hz, 1 H), 7,82 (bd, J= 2Hz, 1 H), 8,42 (bs, 2H).

### EXEMPLE 3

### Composé n°27: 1-[4-(3-trifluorométhyl-4-chloro-phényl)-3,6-dihydro-2H-pyridin-1-yl]-2-[8-(3- pyridin-2-yl)-3,8-diaza-bicyclo[3.2.1]oct-3-yl]-éthanone

En opérant comme décrit dans l'exemple 2, mais en utilisant le composé de la préparation 3 au lieu du composé de la préparation 2 on obtient le composé du titre sous forme de base libre.

RMN (Appareil a). δ (ppm, dmso-d6): 1,77 - 1,98 (m, 4H), 2,40 - 2,67 (m, **), 3,09 (s, 1 H), 3,13 (s, 1 H), 3,68 (m, 1 H), 3,77 (m, 1 H), 4,11 (bs, 1 H), 4,26 - 4,40 (m, 3H), 6,38 (m, 0.5H), 6,42 (m, 0,5H), 7,11 - 7,22 (m, 2H), 7,71 (d, J= 8,5Hz, 1H), 7,76 (bd, J= 8,5Hz, 1H), 7,82 (bd, J= 2Hz, 1H), 7,86 (bs, 1H), 8,19 (m, 1H).

### EXEMPLE 4

### Composé n°28: Méthyl ester de l'acide 6-(3-{2-[4-(4-Chloro-3-trifluorométhyl-phényl)-3,6-dihydro-2H-pyridin-1-yl]-2-oxo-éthyl}-3,8-diaza-bicyclo[3.2.1]oct-8-yl)-nicotinique et son chlorhydrate

En opérant comme décrit dans l'exemple 2, mais en utilisant le composé de la préparation 4 au lieu du composé de la préparation 2 on obtient le composé du titre sous forme de base libre. On dissous dans du diéthyl éther et on ajoute une solution d'isopropanol saturé en HCl et on obtient la formation du chlorhydrate sous forme d'un solide vitreux.

RMN (Appareil a). δ (ppm, dmso-d6): 2,12 (m, 2H), 2,24 (m, 2H), 2,46 - 2,55 (m, **), 2,60 (bs, 1 H), 3,27 (m, 2H), 3,46 - 3,64 (m, 3H), 3,72 (m, 1 H), 3,83 (s, 3H), 4,05 (bs, 1 H), 4,17 (bs, *), 4,20 - 4,36 (m, *), 4,89 (bs, 2H), 6,37 (m, 1 H), 6,97 (d, J= 9,0Hz, 1H), 7,69 - 7,78 (m, 2H), 7,80 (m, 1 H), 8,07 (m, 1 H), 8,71 (m, 1 H), 9,4 - 10,1 (bs, 1 H).

### EXEMPLE 5

### Composé n°29: Acide 6-(3-{2-[4-(4-Chloro-3-trifluorométhyl-phényl)-3,6-dihydro-2H-pyridin-1-yl]-2-oxo-éthyl}-3,8-diaza-bicyclo[3.2.1]oct-8-yl)-nicotinique et son fumarate

On dissous 0,35 g du composé de l'exemple 4 dans 3 ml de solution aqueuse de HCl à 35%. On chauffe à la température de reflux pendant une heure, on lave avec de l'éther éthylique. On ajuste le pH à 5 avec une solution de NaOH et on extrait avec de l'acétate d'éthyle. On sèche et on évapore la phase organique et on obtient 220 mg d'un solide vitreux. On dissous dans l'isopropanol et on ajoute une solution d'acide fumarique dans l'isopropanol. On obtient une précipitation du fumarate que l'on filtre.

On isole 70 mg de produit du titre sous forme de solide blanc.

RMN (Appareil a). δ (ppm, dmso-d6): 1,74 - 2,07 (m, 4H), 2,31 - 2,55 (m, **), 2,58 - 2,76 (m, 3H), 3,09 - 3,36 (m, *), 3,68 (m, 1 H), 3,78 (m, 1 H), 4,12 (bs, 1 H), 4,35 (bs, 1 H), 4,64 (bs, 2H), 6,40 (m, 1 H), 6,64 (s, 2H), 6,76 (m, 1 H), 7,71 (d, J= 8,5Hz, 1 H), 7,76 (m, 1 H), 7,82 (bs, 1 H), 7,91 (m, 1 H), 8,62 (bs, 1 H), 12,1 - 13,4 (m, 2H).

### EXEMPLE 6

### Composé n°53: Méthyl ester de l'acide 3-(1-{2-[8-(5-Fluoro-pyrimidin-2-yl)-3,8-diaza-bicyclo[3.2.1]oct-3-yl]-acétyl}-1,2,5,6-tétrahydro-pyridin-3-yl)-benzoique

En opérant comme décrit dans l'exemple 2, mais en utilisant le composé de la préparation 10 au lieu du composé de la préparation 9, on obtient le composé du titre sous forme de base libre.

RMN (Appareil a). δ (ppm, dmso-d6): 1,66 (m, 2H), 1,78 - 1,99 (m, 2H), 2,23 - 2,35 (m, 2H), 2,35 - 2,46 (m, 2H), 2,57 - 2,75 (m, 2H), 3,19 (s, 2H), 3,61 (m, 1 H), 3,70 (m, 1 H), 3,88 (s, 3H), 4,34 (s, 1H), 4,50 - 4,72 (m, 3H), 6,41 (m, 0.5H), 6,46 (m, 0.5H), 7,54 (m, 1 H), 7,76 (m, 1 H), 7,89 (m, 1H), 7,96 (bs, 0.5H), 8,02 (bs, 0.5H), 8,43 (m, 2H).

Le tableau suivant décrit les exemples obtenus par application et/ou adaptation de méthodes décrites au moyen des réactifs et produits de départ appropriés :

| | | | | | | | | | |
|---|---|---|---|---|---|---|---|---|---|
| 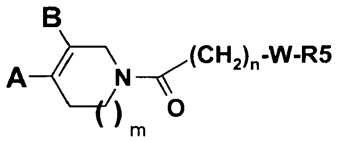 | | | | | | | | | |

| **N** | **A** | **B** | **m** | **W** | **R5** | **n** | **Sel** | **PF** | **LCMS** |
|---|---|---|---|---|---|---|---|---|---|
| **1** | 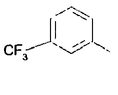 | H | 1 | 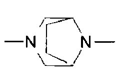 | 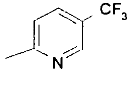 | 1 | Oxalate | 217-219 | MH+ 525 r.t. 14,9 Méthode F |
| **2** | 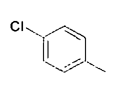 | H | 1 | 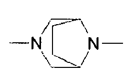 | 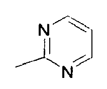 | 1 | - | 181-183 | MH+ 424 r.t. 8,6 Méthode A |
| **3** | 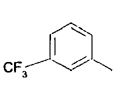 | H | 1 | 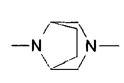 | 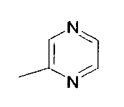 | 1 | Oxalate | - | M+ = 458 r.t.10,9 Méthode E |
| **4** | 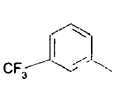 | H | 1 | 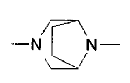 | 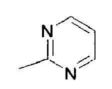 | 1 | HCl | - | M+ = 458 r.t. 8,8 Méthode D |
| **5** | 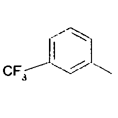 | H | 1 | 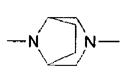 | 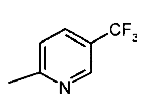 | 1 | Oxalate | - | M+ = 525 r.t. 11,8 Méthode F |
| **6** | 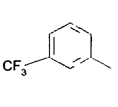 | H | 1 | 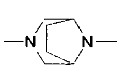 | 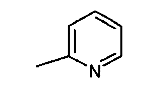 | 1 | Oxalate | 123-126 | MH+ 457 r.t. 8 Méthode C |
| **7** | 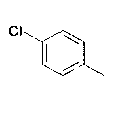 | H | 1 | 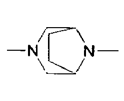 | 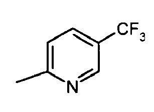 | 1 | Oxalate | 163-164 | MH+ 491 r.t. 9,0 Méthode C |
| **8** | 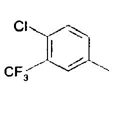 | H | 1 | 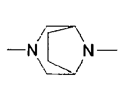 | 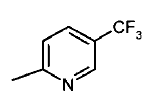 | 1 | Oxalate | 214-215 | MH+ 559 r.t. 7,2 MéthodeA |
| **9** | 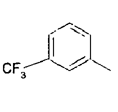 | H | 1 | 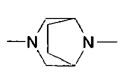 | 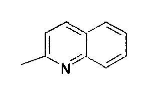 | 1 | - | - | M+ = 507 r.t. 6,1 Méthode A |
| **10** | 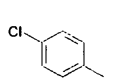 | H | 1 | 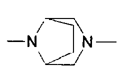 | 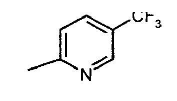 | 1 | Oxalate | - | M+ = 491 r.t. 5,7 Méthode A |
| **12** | 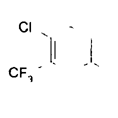 | H | 1 | 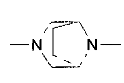 | 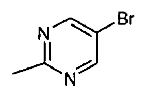 | 1 | - | 147-149 | MH+ 570 r.t. 6,8 Méthode A |
| **13** | 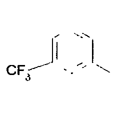 | H | 1 | 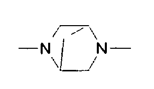 | 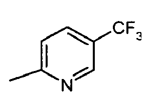 | 1 | Oxalate | - | M+ =511 r.t. 13,0 MéthodeC |
| **14** | 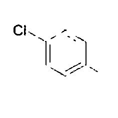 | H | 1 | 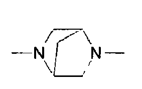 | 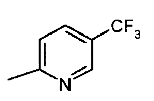 | 1 | Oxalate | 129-131 | MH+ 477 r.t. 8,0 MéthodeG |
| **15** | 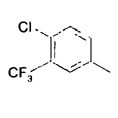 | H | 1 | 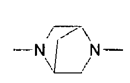 | 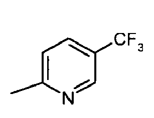 | 1 | Oxalate | - | M+ = 545 r.t. 6,2 Méthode A |
| **16** | 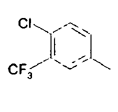 | H | 1 | 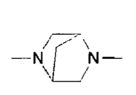 | 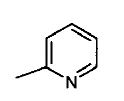 | 1 | Oxalate | 135-137 | MH+ 477 r.t. 8,1 MéthodeC |
| **17** | 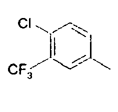 | H | 1 | 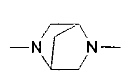 | 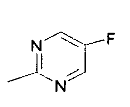 | 1 | HCl | 247-250 | MH+ 496 r.t. 4,7 MéthodeH |
| **18** | 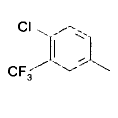 | H | 1 | 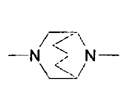 | 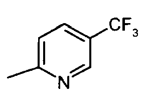 | 1 | Oxalate | - | M+ = 573 r.t. 7,2 Méthode A |
| **19** | 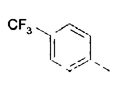 | H | 1 | 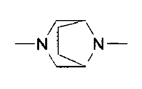 | 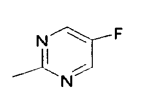 | 1 | - | 154-155 | MH+ 476 r.t. 6,0 Méthode A |
| **20** | 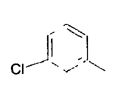 | H | 1 | 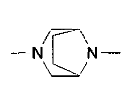 | 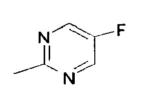 | 1 | - | 146-147 | MH+ 442 r.t. 5,7 Méthode A |
| **21** | 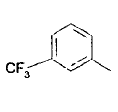 | H | 1 | 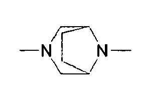 | 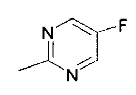 | 1 | HCl | 227-229 | MH+ 476 r.t. 5,9 Méthode A |
| **22** | 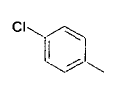 | H | 1 | 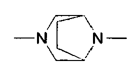 | 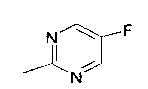 | 1 | - | 157-159 | MH+ 442 r.t. 6,0 Méthode A |
| **23** | 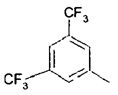 | H | 1 | 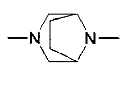 | 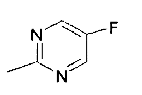 | 1 | HCl | 205-206 | MH+ 544 r.t. 6,9 Méthode A |
| **24** | 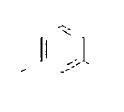 | H | 1 | 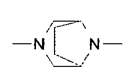 | 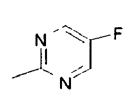 | 1 | - | 133-134 | MH+ 422 r.t. 5,8 Méthode A |
| **25** | 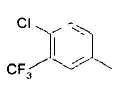 | H | 1 | 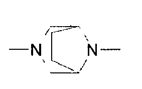 | 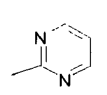 | 1 | - | 120-122 | MH+ 492 r.t. 6,1 Méthode A |
| **26** | 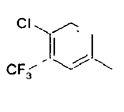 | H | 1 | 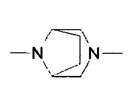 | 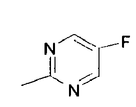 | 1 | HCl | 223-224 | MH+ 510 r.t. 5,6 Méthode A |
| **27** | 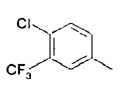 | H | 1 | 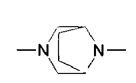 | 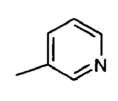 | 1 | - | 136-137 | MH+ 491 r.t. 4,9 Méthode A |
| **28** | 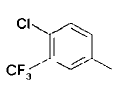 | H | 1 | 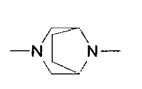 | 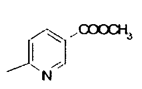 | 1 | HCl | - | M+= 549 r.t. 6,6 Méthode A |
| **29** | 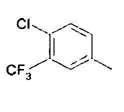 | H | 1 | 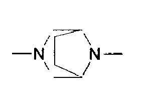 | 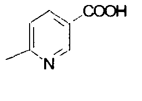 | 1 | fumarate | 165-168 | MH+ 535 r.t. 5,9 Méthode A |
| **30** | 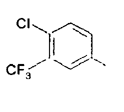 | H | 1 | 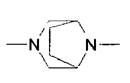 | 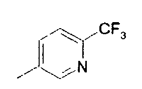 | 1 | - | 161-163 | MH+ 559 r.t. 6,7 Méthode A |
| **31** | 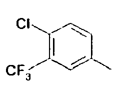 | H | 1 | 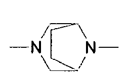 | 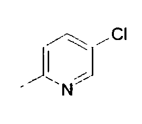 | 1 | oxalate | 219-220 | MH+ 525 r.t. 6,5 Méthode A |
| **32** | 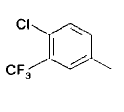 | H | 1 | 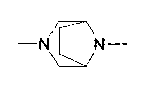 | 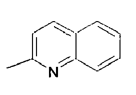 | 1 | oxalate | 135-136 | MH+ 541 r.t. 6,1 Méthode A |
| **33** | 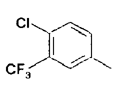 | H | 1 | 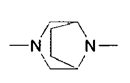 | 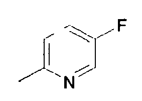 | 1 | oxalate | 203-204 | MH+ 509 r.t. 6,2 Méthode A |
| **34** | 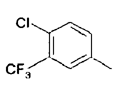 | H | 1 | 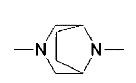 | 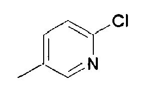 | 1 | oxalate | 212-213 | MH+ 525 r.t. 6,3 Méthode A |
| **35** | 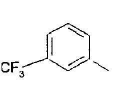 | H | 1 | 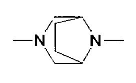 | 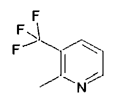 | 1 | oxalate | 190-191 | MH+ 525 r.t. 6,0 Méthode A |
| **36** | 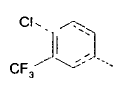 | H | 1 | 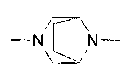 | 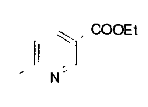 | 1 | oxalate | 210-211 | MH+ 563 r.t. 6,9 Méthode B |
| **37** | 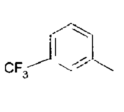 | H | 1 | 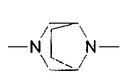 | 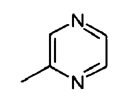 | 1 | oxalate | 220-221 | MH+ 457 r.t. 5,6 Méthode B |
| **38** | 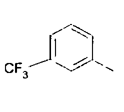 | H | 1 | 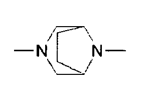 | 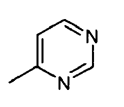 | 1 | fumarate | 161-163 | MH+ 458 r.t. 5,1 Méthode A |
| **39** | 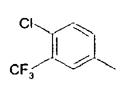 | H | 1 | 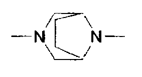 | 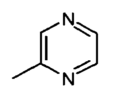 | 1 | oxalate | 150-151 | MH+ 492 r.t. 5,6 Méthode A |
| **40** | 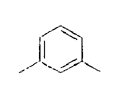 | H | 1 | 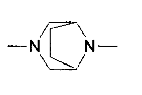 | 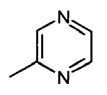 | 1 | oxalate | 162-163 | MH+ 404 r.t. 4,4 Méthode A |
| **41** | 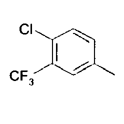 | H | 1 | 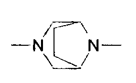 | 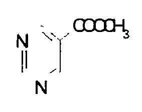 | 1 | - | 152-153 | MH+ 550 r.t. 6,5 Méthode A |
| **42** | 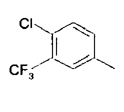 | H | 1 | 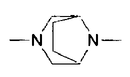 | 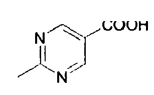 | 1 | - | 192-194 | MH+ 536 r.t. 5,5 MéthodeC |
| **43** | 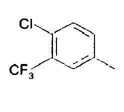 | H | 1 | 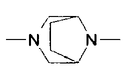 | 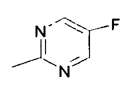 | 2 | oxalate | 140-141 | MH+ 525 r.t. 5,8 Méthode A |
| **44** | 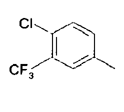 | H | 1 | 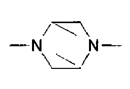 | 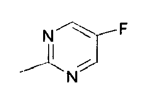 | 1 | - | - | M+= 510 r.t. 6,1 Méthode A |
| **45** | 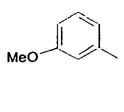 | H | 1 | 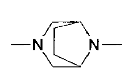 | 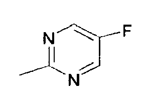 | 1 | oxalate | 171-172 | MH+ 438 r.t. 5,4 Méthode B |
| **46** | 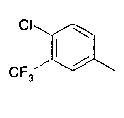 | H | 1 | 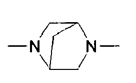 | 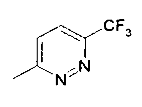 | 1 | fumarate | 205-207 | MH+ 546 r.t. 6,1 Méthode B |
| **47** | 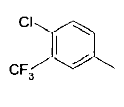 | H | 0 | 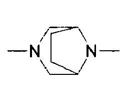 | 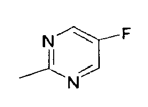 | 1 | - | 200-201 | MH+ 496 r.t. 5.6 Méthode A |
| **48** | 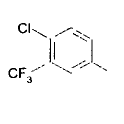 | H | 1 | 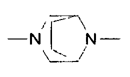 | 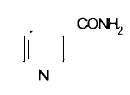 | 1 | - | 241-242 | MH+ 534 r.t. 5,0 Méthode B |
| **49** | 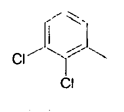 | H | 1 | 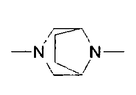 | 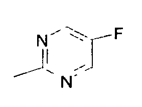 | 1 | - | 127 | MH+ 476 r.t. 5.7 Méthode |
| **50** | 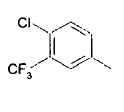 | H | 1 | 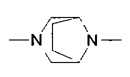 | 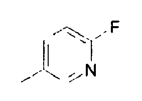 | 1 | oxalate | 160-161 | MH+ 509 r.t. 6,0 Méthode A |
| **51** | 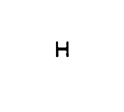 | 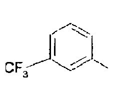 | 1 | 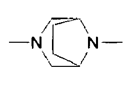 | 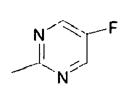 | 1 | - | 156-157 | MH+ 476 r.t. 5,9 Méthode A |
| **52** | 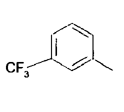 | H | 0 | 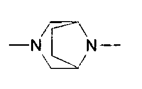 | 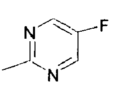 | 1 | - | 195-196 | MH+ 462 r.t. 5,5 Méthode A |
| **53** | 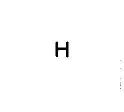 | 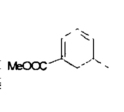 | 1 | 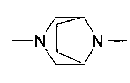 | 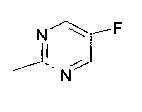 | 1 | - | 140-143 | MH+ 466 r.t. 5,1 Méthode A |
| **54** | 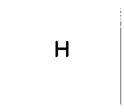 | 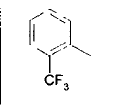 | 1 | 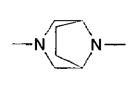 | 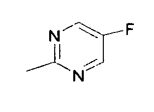 | 1 | oxalate | 215-217 | MH+ 476 r.t. 5,6 Méthode A |
| **55** | 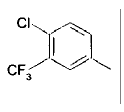 | H | 1 | 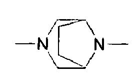 | 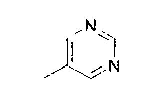 | 1 | HCl | 215-217 | MH+ 492 r.t. 5,6 Méthode A |
| **56** | 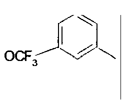 | H | 1 | 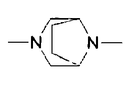 | 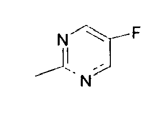 | 1 | - | 135-136 | MH+ 492 r.t. 5,7 Méthode A |
| **57** | 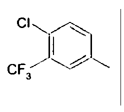 | H | 1 | 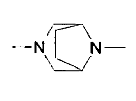 | 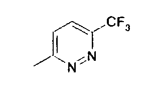 | 1 | - | 161-162 | MH+560 r.t.6,4 MéthodeA |
| **58** | 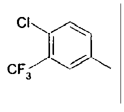 | H | 1 | 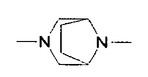 | 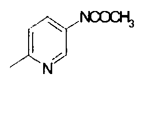 | 1 | | | MH+ 549 |
| **59** | 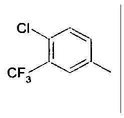 | H | 1 | 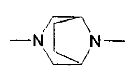 | 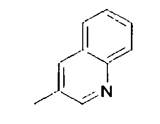 | 1 | - | - | MH+ 541 r.t.5,8 MéthodeA |

Les composés selon l'invention ont fait l'objet d'études biochimiques.

### Culture cellulaire :

La souche SH-SY-5Y (neuroblastome humain) est cultivée classiquement dans un milieu de culture DMEM (de l'anglais Dulbecco's Modified Eagle's Medium) (Gibco BRL, France) contenant du SVF (5%) (sérum de veau foetal) (Boehringer Mannheim, Allemagne), du pyruvate de sodium (1 mM) et de la glutamine (4 mM) dans des flacons de culture recouvert de collagène (Becton Dickinson, France).

La souche mère SK-N-BE (neuroblastome humain) et le clone Bep 75, exprimant de manière stable la forme entière du récepteur p75^{NTR} humain (SK-N-BE Bep 75) sont cultivés classiquement dans un milieu de culture RPMI contenant du SVF (5%), du pyruvate de sodium (1 mM) et de la glutamine (4 mM). Pour les cellules SK-N-BE Bep 75, on ajoute de l'hygromycine (200 µl/20ml milieu) comme agent de sélection.

### Etude de la liaison du ¹²⁵ I NGF au récepteur p75^{NTR}

L'étude de la liaison du NGF (le facteur de croissance neuronale radiomarqué à l'iode-125, Amersham - 2000 Ci/mmol) est réalisée sur une suspension cellulaire de la souche SK-N-BE Bep 75 en accord avec la méthode décrite par Weskamp (Neuron, 1991,6, 649-663). La liaison non spécifique est déterminée par la mesure de la liaison totale après une heure de pré-incubation avec les cellules à 37°C en présence de NGF non-radio marqué (1 µM). La liaison spécifique est calculée par différence entre la mesure de la liaison totale et la mesure de la liaison nonspécifique. Les expériences de compétition sont réalisées en utilisant une concentration en NGF-iodé (¹²⁵I NGF) de 0,3 nM. Les concentrations inhibitrices de 50 %(CI₅₀) de la fixation de ¹²⁵I NGF au récepteur p75^{NTR} des composés selon l'invention sont faibles et varient de 10⁻⁶ à 10⁻¹¹ M.

Les composés de formule (I) présentent une activité dans ce test avec des CI₅₀ qui varient de 10⁻⁶ à 10⁻¹¹M. Par exemple, les composés n°1, 2, 7 et 11 ont montré respectivement une CI₅₀ de 1,35 nM, 0,18 nM, 0,29 nM et 0,98 nM.

Etude de la dimérisation du récepteur p75^{NTR} indépendamment de son ligand L'étude de la dimérisation du récepteur p75^{NTR} est réalisée sur une suspension cellulaire de la souche SK-N-BE Bep 75. Les cellules (2.5 10⁴ cellules/puits) sont disposées dans des puits (plaque de 96 puits) pendant 24 h, puis pré-incubées pendant 1h à 37°C en présence ou non des composés selon l'invention. On ajoute ensuite du surnageant, issu de culture de cellules humaines d'origine rénale HEK293 exprimant, après 48h de transfection, et sécrétant une forme soluble du récepteur p75^{NTR} (partie extracellulaire du récepteur) couplée à une phosphatase alcaline, ce à la concentration finale de 10 nM. La quantification de la liaison spécifique du récepteur soluble p75^{NTR} au récepteur présent sur cellules SK-N-BE Bep 75 est déterminée par la mesure de l'activité enzymatique de la phosphatase alcaline après incubation des cellules pendant 1 heure à 37°C en présence du surnageant. Après filtration et transfert des filtres dans des plaques de 24 puits, l'activité de la phosphatase alcaline est déterminée par ajout de substrat chimioluminescent CDP-Star (ready-to-use, Roche). Les concentrations inhibitrices de 50 %(CI₅₀) de la dimérisation du récepteur p75^{NTR} des composés selon l'invention sont faibles et varient de10⁻⁶ à 10⁻¹¹M.

Les composés de formule (I) présentent une activité dans ce test avec des CI₅₀ qui varient de 10⁻⁶ à 10⁻¹¹M.

Par exemple, les composés n° 1, 3, 8, 11, 27, 28, 29 et 53, ont montré respectivement une CI₅₀ de 23,4 nM, 0,05 nM, 0,68 nM, 0,2 nM,.0,23 nM, 9,84 nM, 0,14 nM et 2,08 nM.

### Mesure de l'apoptose

Les cellules (souches de neuroblastomes humains SH-SY-5Y et SK-N-BE Bep 75) sont installées dans des boîtes de Pétri de 35 mm de diamètre (Biocoat collagenl, (10⁵ cellules/puits)) dans un milieu de culture approprié contenant 5 % de SVF durant 24H. Le milieu de culture est ensuite éliminé, les cellules sont rincées avec du PBS (de l'anglais Dulbecco's Phosphate buffered saline), puis on ajoute soit du milieu frais contenant 5% de SVF, soit du milieu contenant du NGF (à la concentration de 10 ng/ml), soit du peptide beta-amyloïde (Aβ1-40) (à la concentration de 10 µM), ceci en présence ou non des composés selon l'invention. Les taux d'apoptose sont mesurés 48 heures après les traitements dans le cas de la souche SH-SY-5Y, et 24 heures après dans le cas de la souche SK-N-BE Bep 75 par quantification des histones cytoplasmiques associés aux fragments d'ADN (cell death détection ELISA, Boehringer Mannheim, Allemagne). Les taux d'apoptose sont exprimés en quantité d'oligonucléosomes/10⁵ cellules. Chaque valeur correspond à la moyenne de 9 points expérimentaux répartis dans 3 expériences indépendantes.

Les composés de formule (I) présentent une activité inhibitrice de l'apoptose induite par le NGF avec des CI₅₀ qui varient de 10⁻⁶ à 10⁻¹¹M. Par exemple, les composés n° 1, 3, 8, 11, 27 et 29 ont montré respectivement une CI₅₀ de 1,33 nM, 0,067 nM, 2,24 nM, 0,21 nM, 0,088 nM 0,22 nM

Ainsi, la fixation des composés selon l'invention au récepteur p75^{NTR} se traduit, d'une part, au niveau biochimique par l'inhibition de la dimérisation du récepteur induit par les neurotrophines, ou indépendamment du ligand, et, d'autre part, au niveau cellulaire par l'inhibition de l'effet proapoptotique médié par le récepteur p75^{NTR}.

Ainsi, selon un des objets de la présente invention, les composés de formule (I) présentent une activité très intéressante d'inhibition de la dimérisation du récepteur p75^{NTR} indépendamment de son ligand.

Les composés selon l'invention peuvent donc être utilisés pour la préparation de médicaments, en particulier de médicaments destinés à prévenir ou à traiter toute pathologie où le récepteur p75^{NTR} est impliqué, plus particulièrement celles indiquées ci-après.

Les composés selon l'invention peuvent également être utilisés pour prévenir ou traiter toute pathologie où le récepteur p75^{NTR} est impliqué, plus particulièrement celles indiquées ci-après.

Ainsi, selon un autre de ses aspects, l'invention a pour objet des médicaments qui comprennent un composé de formule(I), ou un sel d'addition de ce dernier à un acide pharmaceutiquement acceptable.

Ainsi, les composés selon l'invention peuvent être utilisés, chez l'homme ou chez l'animal, dans le traitement ou la prévention de différentes affections p75^{NTR} dépendantes telles que les maladies neurodégénératives centrales et périphériques comme la démence sénile, l'épilepsie, la maladie d'Alzheimer, la maladie de Parkinson, la chorée d'Huntington, le syndrome de Down, les maladies à prion, l'amnésie, la schizophrénie, la dépression, le désordre bipolaire; la sclérose latérale amyotrophique, la sclérose en plaques ; les affections cardiovasculaires comme les dommages cardiaques post-ischémiques, les cardiomyopathies, l'infarctus du myocarde, l'insuffisance cardiaque, l'ischémie cardiaque, l'infarctus cérébral ; les neuropathies périphériques (d'origine diabétique, traumatisme ou iatrogène) ; les dommages au nerf optique et à la rétine (dégénérescence du pigment rétinien, glaucome) ; l'ischémie rétinale ; la dégénérescence maculaire ; les traumatismes de la moelle épinière et les traumatismes crâniens ; l'athérosclérose ; les sténoses ; les désordres de la cicatrisation ; l'alopécie.

Les composés selon l'invention peuvent également être utilisés dans le traitement des cancers comme celui du poumon, de la thyroïde, du pancréas, de la prostate, de l'intestin grêle et du colon, du sein, dans le traitement des tumeurs, des métastases et des leucémies.

Les composés selon l'invention peuvent également être utilisés dans le traitement des désordres respiratoires comme l'inflammation pulmonaire, l'allergie et l'asthme, la broncho-pneumopathie chronique obstructive.

Les composés selon l'invention peuvent aussi être utilisés dans le traitement de la douleur cutanée (de la peau, des tissus sous-cutanés et organes associés), somatique, viscérale (au niveau du système circulatoire, respiratoire, gastro-intestinal, ou génito-urinaire), et neurologiques.

Les composés selon l'invention peuvent être utilisés dans le traitement des douleurs chroniques neuropathiques et inflammatoires et dans le traitement des maladies auto-immunes comme la polyarthrite rhumatoïde.

Les composés selon l'invention peuvent aussi être utilisés dans le traitement des maladies comme la spondylarthrite ankylosante, le rhumatisme psoriasique, le psoriasis en plaques.

Les composés selon l'invention peuvent égaiement être utilisés dans le traitement des fractures osseuses, dans le traitement ou la prévention des maladies osseuses comme l'ostéoporose.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel peut être administré sous forme unitaire d'administration, en mélange avec des excipients pharmaceutiques classiques, aux animaux et aux êtres humains pour la prévention ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, parentérale telle que transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

La dose de principe actif administrée par jour peut atteindre 0,01 à 100 mg/kg, en une ou plusieurs prises, préférentiellement 0,02 à 50 mg/kg. En général, la dose journalière du composé de l'invention sera la dose efficace la plus faible du composé capable de produire un effet thérapeutique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Composé répondant à la formule (I) : 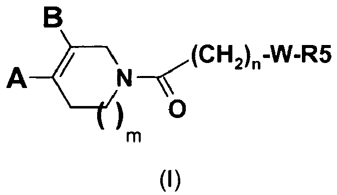 dans laquelle :
- m représente 0 ou 1 ;
- A représente : 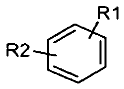 et B représente un atome d'hydrogène ou
A représente un atome d'hydrogène et B représente : 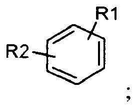
- W- est un hétérocycle azoté choisi parmi : 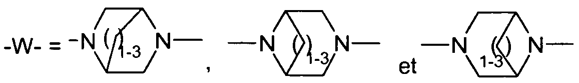
- 1-3 représente 1, 2 ou 3 ;
- n représente 1 ou 2 ;
- R1 représente un atome d'halogène, un groupe (C1-C4)alkyle, un radical trifluorométhyle, un groupe (C1-C4)alkoxy ou un radical trifluorométhoxy ;
- R2 représente un atome d'hydrogène, un atome d'halogène, un groupe (C1-C4)alkyle, un radical trifluorométhyle, un groupe (C1-C4)alkoxy, un radical trifluorométhoxy, un groupe COOR ou un groupe CONH2 ;
- R5 représente un groupe de formule : 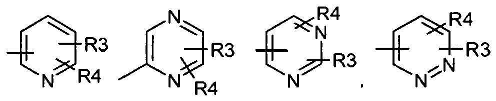 ou 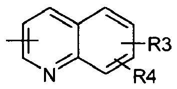 dans lesquels R3 et R4 situés sur l'une quelconque des positions disponibles, représentent indépendamment un atome d'hydrogène, un atome d'halogène, un groupe (C1-C4)alkyle, (C1-C4)alkoxy, un radical trifluorométhyle, trifluorométhoxy un cyano, un groupe COOH, COOalkyle, CONH2, CONR6R7 ou NHCOR ;
- R, R6 et R7 représentent un groupe C1-C6 alkyle;
à l'état de base ou de sel d'addition à un acide.

2. Composé selon la revendication 1 tel que R2 est un atome d'hydrogène, un radical trifluorométhyle, un groupe COOR ou un groupe CONH2; à l'état de base ou de sel d'addition à un acide.

3. Composé selon la revendication 1 ou 2 tel que R5 représente un groupe de formule : 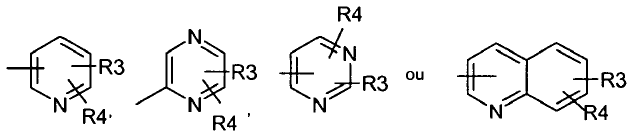 où R3 et R4 représentent indépendamment un atome d'hydrogène, un halogène, un radical trifluorométhyle, CONH2, COOH ou NHCOCH3; à l'état de base ou de sel d'addition à un acide.

4. Composé selon la revendication 1 tel que :
- m représente 1 ;
- A représente : 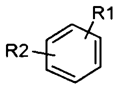 et B représente un atome d'hydrogène ;
- W- est un hétérocycle azoté choisi parmi : 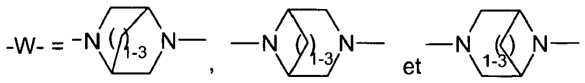
- n représente 1 ou 2 ;
- R1 représente un atome d'halogène, un groupe (C1-C4)alkyle, un radical trifluorométhyle, un groupe (C1-C4)alkoxy ou un radical trifluorométhoxy ;
- R2 représente un atome d'hydrogène, un atome d'halogène, un groupe (C1-C4)alkyle, un radical trifluorométhyle, un groupe (C1-C4)alkoxy ou un radical trifluorométhoxy ;
- R5 représente un groupe de formule : 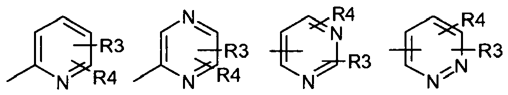 ou 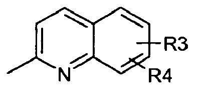
dans lesquels R3 et R4, situés sur l'une quelconque des positions disponibles, représentent indépendamment un atome d'hydrogène, un atome d'halogène, un groupe (C1-C4)alkyle, (C1-C4)alkoxy, un radical trifluorométhyle, trifluorométhoxy ou cyano, un groupe COOH ou COOalkyle ;
à l'état de base ou de sel d'addition à un acide.

5. Composé selon l'une quelconque des revendications précédentes tel que W est un groupe de formule choisi parmi: 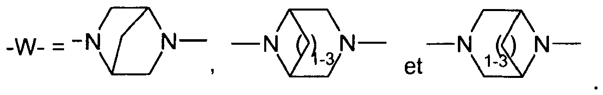 à l'état de base ou de sel d'addition à un acide.

6. Composé selon l'une quelconque des revendications précédentes tel que n = 1 ; à l'état de base ou de sel d'addition à un acide.

7. Composé selon l'une quelconque des revendications précédentes tel que R1 est un atome d'halogène ou un radical trifluorométhyle ; à l'état de base ou de sel d'addition à un acide.

8. Composé selon l'une quelconque des revendications précédentes tel que R2 est un atome d'hydrogène ou un radical trifluorométhyle ; à l'état de base ou de sel d'addition à un acide.

9. Composé selon l'une quelconque des revendications précédentes tel que R5 représente un groupe de formule : 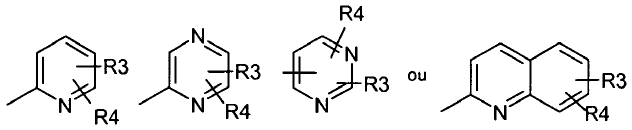 où R3 ou R4 représentent indépendamment un atome d'hydrogène, d'halogène ou un radical trifluorométhyle ; à l'état de base ou de sel d'addition à un acide.

10. Composé selon l'une quelconque des revendications précédentes choisi parmi les composés suivants :
- Composé n°1 : 1-[4-(3-Trifluorométhyl-phényl)-3,6-dihydro-2H-pyridin-1-yl]-2-[8-(5-trifluorométhyl-pyridin-2-yl)-3,8-diaza-bicyclo[3.2.1]oct-3-yl]-éthanone ;
- Composé n°2: 1-[4-(4-Chloro-phényl)-3,6-dihydro-2H-pyridin-1-yl]-2-(8-pyrimidin-2-yl-3,8-diaza-bicyclo[3.2.1]oct-3-yl)-éthanone ;
- Composé n°3: 2-(3-Pyrazin-2-yl-3,8-diaza-bicyclo[3.2.1]oct-8-yl)-1-[4-(3-trifluorométhyl-phényl)-3,6-dihydro-2H-pyridin-1-yl]-éthanone ;
- Composé n°4: 2-(8-Pyrimidin-2-yl-3,8-diaza-bicyclo[3.2.1]oct-3-yl)-1-[4-(3-trifiuorométhyl-phényl)-3,6-dihydro-2H-pyridin-1-yl]-éthanone ;
- Composé n°5: 1-[4-(3-trifluorométhyl-phényl)-3,6-dihydro-2H-pyridin-1-yl]-2-[3-(5-trifluorométhyl-pyridin-2-yl)-3,8-diaza-bicyclo[3.2.1]oct-8-yl]-éthanone ;
- Composé n°6: 2-(8-Pyridin-2-yl-3,8-diaza-bicyclo[3.2.1]oct-3-yl)-1-[4-(3-trifluorométhyl-phényl)-3,6-dihydro-2H-pyridin-1-yl]-éthanone;
- Composé n°7: 1-[4-(4-chloro-phényl)-3,6-dihydro-2H-pyridin-1-yl]-2-[8-(5-trifluorométhyl-pyridin-2-yl)-3,8-diaza-bicyclo[3.2.1]oct-3-yl]-éthanone ;
- Composé n°8: 1-[4-(4-chloro-3-trifluorométhyl-phényl)-3,6 dihydro-2H-pyridin-1-yl]-2-[8-(5-trifluorométhyl-pyridin-2-yl)-3,8-diaza-bicyclo[3.2.1]oct-3-yl]-éthanone ;
- Composé n°9: 2-(8-Quinolin-2-yl-3,8-diaza-bicyclo[3.2.1]oct-3-yl)-1-[4-(3-trifluorométhyl-phényl)-3,6-dihydro-2H-pyridin-1-yl]-éthanone;
- Composé n°10: 1-[4-(4-chloro-phényl)-3,6-dihydro-2H-pyridin-1-yl]-2-[3-(5-trifluorométhyl-pyridin-2-yl)-3,8-diaza-bicyclo[3.2.1]oct-8-yl]-éthanone ;
- Composé n°11: 1-[4-(4-chloro-3-trifluorométhyl-phényl)-3,6-dihydro-2H-pyridin-1-yl]-2-[8-(5-fluoro-pyrimidin-2-yl)-3,8-diaza-bicyclo[3.2.1]oct-3-yl]-éthanone ;
- Composé n°12: 2-[8-(5-Bromo-pyrimidin-2-yl)-3,8-diaza-bicyclo[3.2.1]oct-3-yl]-1-[4-(4-chloro-3-trifluorométhyl-phényl)-3,6-dihydro-2H-pyridin-1-yl]-éthanone;
- Composé n°13: 1-[4-(3-trifluorométhyl-phényl)-3,6 dihydro-2H-pyridin-1-yl]-2-[5-(5-trifluorométhyl-pyridin-2-yl)-2,5-diaza-bicyclo[2.2.1]hept-2-yl]-éthanone ;
- Composé n°14: 1-[4-(4-Chloro-phényl)-3,6-dihydro-2H-pyridin-1-yl]-2-[5-(5-trifluorométhyl-pyridin-2-yl)-2,5-diaza-bicyclo[2.2.1]hept-2-yl]-éthanone ;
- Composé n°15: 1-[4-(4-Chloro-3-trifluorométhyl-phényl)-3,6-dihydro-2H-pyridin-1-yl]-2-[5-(5-trifluorométhyl-pyridin-2-yl)-2,5-diaza-bicyclo[2.2.1]hept-2-yl]-éthanone ;
- Composé n°16: 1-[4-(4-Chloro-3-trifluorométhyl-phényl)-3,6-dihydro-2H-pyridin-1-yl]-2-(5-pyridin-2-yl-2,5-diaza-bicyclo[2.2.1]hept-2-yl)-éthanone;
- Composé n°17: 1-[4-(4-Chloro-3-trifluorométhyl-phényl)-3,6-dihydro-2H-pyridin-1-yl]-2-[5-(5-fluoro-pyrimidin-2-yl)-2,5-diaza-bicyclo[2.2.1]hept-2-yl]-éthanone;
- Composé n°18: 1-[4-(4-Chloro-3-trifluorométhyl-phényl)-3,6-dihydro-2H-pyridin-1-yl]-2-[9-(5-trifluorométhyl-pyridin-2-yl)-3,9-diaza-bicyclo[3.3.1]non-3-yl]-éthanone;
- Composé n°19: 2-[8-(5-Fluoro-pyrimidin-2-yl)-3,8-diaza-bicyclo[3.2.1]oct-3-yl]-1-[4-(4-trifluorométhyl-phényl)-3,6-dihydro-2H-pyridin-1-yl]-éthanone ;
- Composé n°20: 1-[4-(3-chloro-phényl)-3,6-dihydro-2H-pyridin-1-yl]-2-[8-(5-fluoro-pyrimidin-2-yl)-3,8-diaza-bicyclo[3.2.1]oct-3-yl]-éthanone ;
- Composé n°21 :2-[8-(5-Fluoro-pyrimidin-2-yl)-3,8-diaza-bicyclo[3.2.1]oct-3-yl]-1-[4-(3-trifluorométhyl-phényl)-3,6-dihydro-2H-pyridin-1-yl]-éthanone;
- Composé n°22: 1-[4-(4-chlorol-phényl)-3,6-dihydro-2H-pyridin-1-yl]-2-[8-(5-fluoropyrimidin-2-yl)-3,8-diaza-bicyclo[3.2.1]oct-3-yl]-éthanone ;
- Composé n°23: 1-[4-(3,5-Bis-trifluorométhyl-phényl)-3,6-dihydro-2H-pyridin-1-yl]-2-[8-(5-fluoro-pyrimidin-2-yl)-3,8-diaza-bicyclo[3.2.1]oct-3-yl]-éthanone;
- Composé n°24: 2-[8-(5-Fluoro-pyrimidin-2-yl)-3,8-diaza-bicyclo[3.2.1]oct-3-yl]-1-(4-m-tolyl-3,6-dihydro-2H-pyridin-1-yl)-éthanone;
- Composé n°25 : 1-[4-(4-chloro-3-trifluorométhyl -phényl)-3,6- dihydro-2H-pyridin-1-yl]-2-[8-(pyrimidin-2-yl)-3,8-diaza-bicyclo[3.2.1]oct-3-yl]-éthanone ;
- Composé n°26: 1-[4-(4-Chloro-3-trifluorométhyl-phényl)-3,6-dihydro-2H-pyridin-1-yl]-2-[3-(5-fluoro-pyrimidin-2-yl)-3,8-diaza-bicyclo[3.2.1]oct-8-yl]-éthanone ;
- Composé n°27: 1-[4-(4-Chloro-3-trifluorométhyl-phényl)-3,6-dihydro-2H-pyridin-1-yl]-2-(8-pyridin-3-yl-3,8-diaza-bicyclo[3.2.1]oct-3-yl)-éthanone;
- Composé n°28: Méthyl ester de l'acide 6-(3-{2-[4-(4-Chloro-3-trifluorométhyl-phényl)-3,6-dihydro-2H-pyridin-1-yl]-2-oxo-éthyl}-3,8-diaza-bicyclo[3.2.]oct-8-yl)-nicotinique ;
- Composé n°29 : Acide 6-(3-{2-[4-(4-Chloro-3-trifluorométhyl-phényl)-3,6-dihydro-2H-pyridin-1-yl]-2-oxo-éthyl}-3,8-diaza-bicyclo[3.2.1]oct-8-yl)-nicotinique ;
- Composé n°30: 1-[4-(4-Chloro-3-trifluorométhyl-phényl)-3,6-dihydro-2H-pyridin-1-yl]-2-[8-(6-trifluorométhyl-pyridin-3-yl)-3,8-diaza-bicyclo[3.2.1]oct-3-yl]-éthanone ;
- Composé n°31: 2-[8-(5-Chloro-pyridin-2-yl)-3,8-diaza-bicyclo[3.2.1]oct-3-yl]-1-[4-(4-chloro-3-trifluorométhyl-phényl)-3,6-dihydro-2H-pyridin-1-yl]-éthanone ;
- Composé n°32: 1-[4-(4-Chloro-3-trifluorométhyl-phényl)-3,6-dihydro-2H-pyridin-1-yl]-2-(8-quinolin-2-yl-3,8-diaza-bicyclo[3.2.1]oct-3-yl)-éthanone ;
- Composé n°33: 1-[4-(4-Chloro-3-trifluorométhyl-phényl)-3,6-dihydro-2H-pyridin-1-yl]-2-[8-(5-fluoro-pyridin-2-yl)-3,8-diaza-bicyclo[3.2.1]oct-3-yl]-éthanone ;
- Composé n°34: 2-[8-(6-Chloro-pyridin-3-yl)-3,8-diaza-bicyclo[3.2.1]oct-3-yl]-1-[4-(4-chloro-3-trifluorométhyl-phényl)-3,6-dihydro-2H-pyridin-1-yl]-éthanone ;
- Composé n°35: 1-[4-(3-Trifluorométhyl-phényl)-3,6-dihydro-2H-pyridin-1-yl]-2-[8-(3-trifluorométhyl-pyridin-2-yl)-3,8-diaza-bicyclo[3.2.1]oct-3-yl]-éthanone ;
- Composé n°36: éthyl ester de l'acide 6-(3-{2-[4-(4-Chloro-3-trifluorométhylphényl)-3,6-dihydro-2H-pyridin-1-yl]-2-oxo-éthyl}-3,8-diaza-bicyclo[3.2.1]oct-8-yl)-nicotinique ;
- Composé n°37: 2-(8-Pyrazin-2-yl-3,8-diaza-bicyclo[3.2.1]oct-3-yl)-1-[4-(3-trifluorométhyl-phényl)-3,6-dihydro-2H-pyridin-1-yl]-éthanone ;
- Composé n°38: 2-(8-Pyrimidin-4-yl-3,8-diaza-bicyclo[3.2.1]oct-3-yl)-1-[4-(3-trifluorométhyl-phényl)-3,6-dihydro-2H-pyridin-1-yl]-éthanone ;
- Composé n°39: 1-[4-(4-Chloro-3-trifluorométhyl-phényl)-3,6-dihydro-2H-pyridin-1-yl]-2-(8-pyrazin-2-yl-3,8-diaza-bicyclo[3.2.1]oct-3-yl)-éthanone ;
- Composé n°40: 2-(8-Pyrazin-2-yl-3,8-diaza-bicyclo[3.2.1]oct-3-yl)-1-(4-m-tolyl-3,6-dihydro-2H-pyridin-1-yl)-éthanone ;
- Composé n°41: méthyl ester de l'acide 2-(3-{2-[4-(4-Chloro-3-trifluorométhylphényl)-3,6-dihydro-2H-pyridin-1-yl]-2-oxo-éthyl}-3,8-diaza-bicyclo[3.2.1]oct-8-yl)-pyrimidine-5-carboxylique ;
- Composé n°42: acide 2-(3-{2-[4-(4-Chloro-3-trifluorométhyl-phényl)-3,6-dihydro-2H-pyridin-1-yl]-2-oxo-éthyl}-3,8-diaza-bicyclo[3.2.1]oct-8-yl)pyrimidine-5-carboxylique ;
- Composé n°43: 1-[4-(4-Chloro-3-trifluorométhyl-phényl)-3,6-dihydro-2H-pyridin-1-yl]-3-[8-(5-fluoro-pyrimidin-2-yl)-3,8-diaza-bicyclo[3.2.1]oct-3-yl]-propan-1-one ;
- Composé n°44: 1-[4-(4-Chloro-3-trifluorométhyl-phényl)-3,6-dihydro-2H-pyridin-1-yl]-2-[5-(5-fluoro-pyrimidin-2-yl)-2,5-diaza-bicyclo[2.2.2]oct-2-yl]-éthanone ;
- Composé n°45: 2-[8-(5-fluoro-pyrimidin-2-yl)-3,8-diaza-bicyclo[3.2.1]oct-3-yl]-1-[4-(3-méthoxy-phényl)-3,6-dihydro-2H-pyridin-1-yl]-éthanone ;
- Composé n°46: 1-[4-(4-Chloro-3-trifluorométhyl-phényl)-3,6-dihydro-2H-pyridin-1-yl]-2-[5-(6-trifluorométhyl-pyridazin-3-yl)-2,5-diaza-bicyclo[2.2.1]hept-2-yl]-éthanone ;
- Composé n°47: 2-[8-(5-Fluoro-pyrimidin-2-yl)-3,8-diaza-bicyclo[3.2.1]oct-3-yt]-1-[3-(3-trifluorométhyl-4-chloro-phényl)-2,5-dihydro-pyrrol-1-yl]-éthanone;
- Composé n°48: 6-(3-{2-[4-(4-Chloro-3-trifluorométhyl-phényl)-3,6-dihydro-2H-pyridin-1-yl]-2-oxo-éthyl}-3,8-diaza-bicyclo[3.2.1]oct-8-yl)nicotinamide ;
- Composé n°49: 2-[8-(5-Fluoro-pyrimidin-2-yl)-3,8-diaza-bicyclo[3.2.1]oct-3-yl]-1-[4-(2,3-dichloro-phényl)-3,6-dihydro-2H-pyridin-1-yl]-éthanone ;
- Composé n°50: 1-[4-(4-Chloro-3-trifluorométhyl-phényl)-3,6-dihydro-2H-pyridin-1-yl]-2-[8-(6-fluoro-pyridin-3-yl)-3,8-diaza-bicyclo[3.2.1]oct-3-yl]-éthanone ;
- Composé n°51: 2-[8-(5-Fluoro-pyrimidin-2-yl)-3,8-diaza-bicyclo[3.2.1]oct-3-yl]-1-[5-(3-trifluorométhyl-phényl)-3,6-dihydro-2H-pyridin-1-yl]-éthanone ;
- Composé n°52: 2-[8-(5-Fluoro-pyrimidin-2-yl)-3,8-diaza-bicydo[3.2.1]oct-3-yl]-1-[3-(3-trifluorométhyl-phényl)-2,5-dihydro-pyrrol-1-yl]-éthanone ;
- Composé n°53: Méthyl ester de l'acide 3-(1-{2-[8-(5-Fluoro-pyrimidin-2-yl)-3,8-diaza-bicyclo[3.2.1]oct-3-yl]-acétyl}-1,2,5,6-tétrahydro-pyridin-3-yl)-benzoique ;
- Composé n°54: 2-[8-(5-Fluoro-pyrimidin-2-yl)-3,8-diaza-bicyclo[3.2.1]oct-3-yl]-1-[5-(2-trifluorométhyl-phényl)-3,6-dihydro-2H-pyridin-1-yl]-éthanone ;
- Composé n°55: 1-[4-(4-Chloro-3-trifluorométhyl-phényl)-3,6-dihydro-2H-pyridin-1-yl]-2-(8-pyrimidin-5-yl-3,8-diaza-bicyclo[3.2.1]oct-3-yl)-éthanone ;
- Composé n°56: 2-[8-(5-Fluoro-pyrimidin-2-yl)-3,8-diaza-bicyclo[3.2.1]oct-3-yl]-1-[4-(3-trifluorométhoxyl-phényl)-3,6-dihydro-2H-pyridin-1-yl]-éthanone;
- Composé n°57: 1-[4-(4-Chloro-3-trifluorométhyl-phényl)-3,6-dihydro-2H-pyridin-1-yl]-2-[5-(6-trifluorométhyl-pyridazin-3-yl)-2,5-diaza-bicyclo[2.2.2]oct-2-yl]-éthanone ;
- Composé n°58: N-[6-(3-{2-[4-(4-Chloro-3-trifluorométhyl-phényl)-3,6-dihydro-2H-pyridin-1-yl]-2-oxo-éthyl}-3,8-diaza-bicyclo[3.2.1]oct-8-yl)-pyridin-3-yl]-acétamide ;
- Composé n°59: 2-(8-Quinolin-3-yl-3,8-diaza-bicyclo[3.2.1]oct-3-yl)-1-[4-(3-trifluorométhy-14-chloro -phényl)-3,6-dihydro-2H-pyridin-1-yl]-éthanone;
à l'état de base ou de sel d'addition à un acide.

11. Procédé de préparation d'un composé de formule (I) selon l'une quelconque des revendications 1 à 10, **caractérisé en ce que** l'on fait réagir un composé de formule (II) : 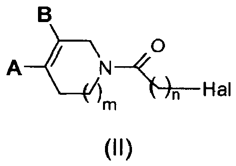 dans laquelle A, B, m et n sont définis selon l'une quelconque des revendications 1 à 10 et Hal représente un atome d'halogène,
et un composé de formule générale (III) :
H-W-R5 (III)
dans laquelle W et R5 sont définis selon l'une quelconque des revendications 1 à 10.

12. Composé de formule (II) : 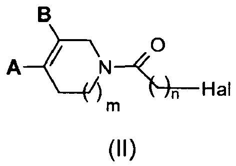 dans laquelle A, B, m et n sont définis selon l'une quelconque des revendications 1 à 10 et Hal représente un atome d'halogène ;
à l'exception du 2-Chloro-1-[4-(2-methoxyphényl)-3,6-dihydro-2H-pyridin-1-yl]-éthanone et du 2-Chloro-1-[4-(4-bromophényl)-3,6-dihydro-2H-pyridin-1-yl]-éthanone ; à l'état de base ou de sel d'addition à un acide.

13. Médicament, **caractérisé en ce qu'**il comprend un composé de formule (I) selon l'une quelconque des revendications 1 à 10, ou un sel d'addition de ce composé à un acide pharmaceutiquement acceptable.

14. Composition pharmaceutique, **caractérisée en ce qu'**elle comprend un composé de formule (I) selon l'une quelconque des revendications 1 à 10, ou un sel pharmaceutiquement acceptable, ainsi qu'au moins un excipient pharmaceutiquement acceptable.

15. Composé selon l'une quelconque des revendications 1 à 10 pour la préparation d'un médicament destiné à la prévention ou le traitement de maladies neurodégénératives centrales et périphériques, la démence sénile, l'épilepsie, la maladie d'Alzheimer, la maladie de Parkinson, la chorée d'Huntington, le syndrome de Down, les maladies à prion, l'amnésie, la schizophrénie, la dépression, le désordre bipolaire, la sclérose latérale amyotrophique, la sclérose en plaques, les affections cardiovasculaires, les dommages cardiaques post-ischémiques, les cardiomyopathies, l'infarctus du myocarde, l'insuffisance cardiaque, l'ischémie cardiaque, l'infarctus cérébral ; les neuropathies périphériques, les dommages au nerf optique et à la rétine, dégénérescence du pigment rétinien, glaucome, l'ischémie rétinale , la dégénérescence maculaire, les traumatismes de la moelle épinière, les traumatismes crâniens, l'athérosclérose, les sténoses, les désordres de la cicatrisation, l'alopécie, les cancers, les tumeurs, les métastases, les leucémies, les désordres respiratoires, l'inflammation pulmonaire, l'allergie, l'asthme, la broncho-pneumopathie chronique obstructive, la douleur cutanée, somatique, viscérale, et neurologique, les douleurs chroniques neuropathiques et inflammatoires, les maladies auto-immunes, la polyarthrite rhumatoïde, la spondylarthrite ankylosante, le rhumatisme psoriasique, le psoriasis en plaques, les fractures osseuses, Jes maladies osseuses, l'ostéoporose.

16. Composé pour la préparation d'un médicament selon la revendication 15 ayant la capacité d'inhiber la dimérisation du récepteur p75^{NTR} indépendamment de son ligand.

## Claims

1. Compound corresponding to the formula (I): 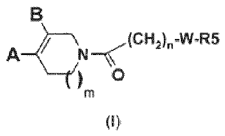 in which:
- m represents 0 or 1;
- A represents: 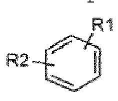 and B represents a hydrogen atom
or
A represents a hydrogen atom and B represents: 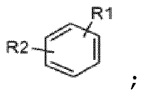
- W- is a nitrogenous heterocycle chosen from: 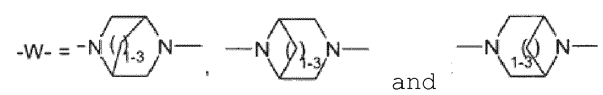
- 1-3 represents 1, 2 or 3;
- n represents 1 or 2;
- R1 represents a halogen atom, a (C₁-C₄)alkyl group, a trifluoromethyl radical, a (C₁-C₄)alkoxy group or a trifluoromethoxy radical;
- R2 represents a hydrogen atom, a halogen atom, a (C₁-C₄) alkyl group, a trifluoromethyl radical, a (C₁-C₄) - alkoxy group, a trifluoromethoxy radical, a COOR group or a CONH₂ group;
- R5 represents a group of formula: 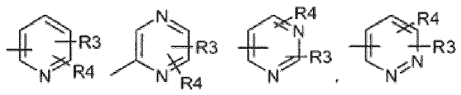 or 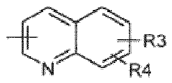 in which R3 and R4, located on any one of the available positions, independently represent a hydrogen atom, a halogen atom, a (C₁-C₄) alkyl group, a (C₁-C₄) alkoxy group, a trifluoromethyl radical, a trifluoromethoxy radical, a cyano radical, a COOH group, a COOalkyl group, a CONH₂ group, a CONR6R7 group or an NHCOR group;
- R, R6 and R7 represent a C₁-C₆ alkyl group;
in the form of the base or of an addition salt with an acid.

2. Compound according to Claim 1, such that R2 is a hydrogen atom, a trifluoromethyl radical, a COOR group or a CONH₂ group; in the form of the base or of an addition salt with an acid.

3. Compound according to Claim 1 or 2, such that R5 represents a group of formula: 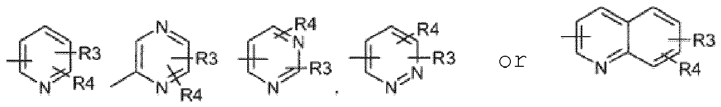 where R3 and R4 independently represent a hydrogen atom, a halogen atom, a trifluoromethyl radical, a CONH₂ radical, a COOH radical or an NHCOCH₃ radical; in the form of the base or of an addition salt with an acid.

4. Compound according to Claim 1, such that:
- m represents 1;
- A represents: 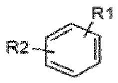 and B represents a hydrogen atom;
- W- is a nitrogenous heterocycle chosen from: 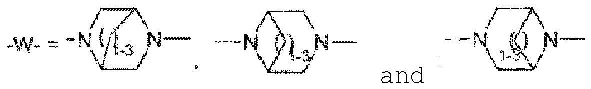
- n represents 1 or 2;
- R1 represents a halogen atom, a (C₁-C₄) alkyl group, a trifluoromethyl radical, a (C₁-C₄)alkoxy group or a trifluoromethoxy radical;
- R2 represents a hydrogen atom, a halogen atom, a (C₁-C₄)alkyl group, a trifluoromethyl radical, a (C₁-C₄)alkoxy group or a trifluoromethoxy radical;
- R5 represents a group of formula: 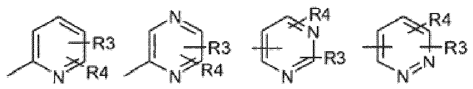
or 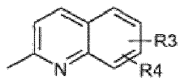 in which R3 and R4, located on any one of the available positions, independently represent a hydrogen atom, a halogen atom, a (C₁-C₄) alkyl group, a (C₁-C₄) alkoxy group, a trifluoromethyl radical, a trifluoromethoxy radical, a cyano radical, a COOH group or a COOalkyl group;
in the form of the base or of an addition salt with an acid.

5. Compound according to any one of the preceding claims, such as W is a group with the formula chosen from: 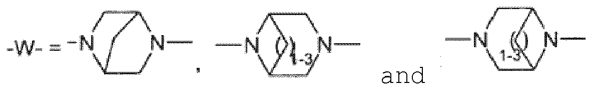 in the form of the base or an addition salt with an acid.

6. Compound according to any one of the preceding claims, such that n = 1; in the form of the base or of an addition salt with an acid.

7. Compound according to any one of the preceding claims, such that R1 is a halogen atom or a trifluoromethyl radical; in the form of the base or of an addition salt with an acid.

8. Compound according to any one of the preceding claims, such that R2 is a hydrogen atom or a trifluoromethyl radical; in the form of the base or of an addition salt with an acid.

9. Compound according to any one of the preceding claims, such that R5 represents a group of formula: 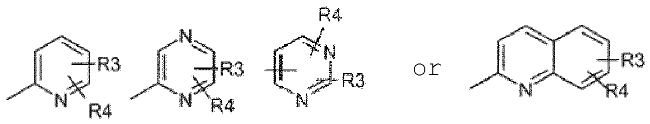 where R3 and R4 independently represent a hydrogen atom, a halogen atom or a trifluoromethyl radical; in the form of the base or of an addition salt with an acid.

10. Compound according to any one of the preceding claims, chosen from the following compounds:
- Compound No. 1: 1-[4-(3-(Trifluoromethyl)phenyl)-3,6-dihydro-2H-pyridin-1-yl]-2-[8-(5-(trifluoromethyl)-pyridin-2-yl)-3,8-diazabicyclo[3.2.1]oct-3-yl]-ethanone;
- Compound No. 2: 1-[4-(4-Chlorophenyl)-3,6-dihydro-2H-pyridin-1-yl]-2-(8-(pyrimidin-2-yl)-3,8-diazabicyclo-[3.2.1]oct-3-yl)ethanone;
- Compound No. 3: 2-(3-(Pyrazin-2-yl)-3,8-diazabicyclo-[3.2.1]oct-8-yl)-1-[4-(3-(trifluoromethyl)phenyl)-3,6-dihydro-2H-pyridin-1-yl]ethanone;
- Compound No. 4: 2-(8-(Pyrimidin-2-yl)-3,8-diazabicyclo[3.2.1]oct-3-yl)-1-[4-(3-(trifluoromethyl)-phenyl)-3,6-dihydro-2H-pyridin-1-yl]ethanone;
- Compound No. 5: 1-[4-(3-(Trifluoromethyl)phenyl)-3,6-dihydro-2H-pyridin-1-yl]-2-[3-(5-(trifluoromethyl)-pyridin-2-yl)-3,8-diazabicyclo[3.2.1]oct-8-yl]-ethanone;
- Compound No. 6: 2-(8-(Pyridin-2-yl)-3,8-diazabicyclo[3.2.1]oct-3-yl)-1-[4-(3-(trifluoromethyl)-phenyl)-3,6-dihydro-2H-pyridin-1-yl]ethanone;
- Compound No. 7: 1-[4-(4-Chlorophenyl)-3,6-dihydro-2H-pyridin-1-yl]-2-[8-(5-(trifluoromethyl)pyridin-2-yl)-3,8-diazabicyclo[3.2.1]oct-3-yl]ethanone;
- Compound No. 8: 1-[4-(4-Chloro-3-(trifluoromethyl)-phenyl)-3,6-dihydro-2H-pyridin-1-yl]-2-[8-(5-(trifluoromethyl)pyridin-2-yl)-3,8-diazabicyclo[3.2.1]-oct-3-yl]ethanone;
- Compound No. 9: 2-(8-(Quinolin-2-yl)-3,8-diazabicyclo[3.2.1]oct-3-yl)-1-[4-(3-(trifluoromethyl)-phenyl)-3,6-dihydro-2H-pyridin-1-yl]ethanone;
- Compound No. 10: 1-[4-(4-Chlorophenyl)-3,6-dihydro-2H-pyridin-1-yl]-2-[3-(5-(trifluoromethyl)pyridin-2-yl)-3,8-diazabicyclo[3.2.1]oct-8-yl]ethanone;
- Compound No. 11: 1-[4-(4-Chloro-3-(trifluoromethyl)-phenyl)-3,6-dihydro-2H-pyridin-1-yl]-2-[8-(5-fluoropyrimidin-2-yl)-3,8-diazabicyclo[3.2.1]oct-3-yl]-ethanone;
- Compound No. 12: 2-[8-(5-Bromopyrimidin-2-yl)-3,8-diazabicyclo[3.2.1]oct-3-yl]-1-[4-(4-chloro-3-(trifluoromethyl)phenyl)-3,6-dihydro-2H-pyridin-1-yl]-ethanone;
- Compound No. 13: 1-[4-(3-(Trifluoromethyl)phenyl)-3,6-dihydro-2H-pyridin-1-yl]-2-[5-(5-(trifluoromethyl)pyridin-2-yl)-2,5-diazabicyclo[2.2.1]hept-2-yl]ethanone;
- Compound No. 14: 1-[4-(4-Chlorophenyl)-3,6-dihydro-2H-pyridin-1-yl]-2-[5-(5-(trifluoromethyl)pyridin-2-yl)-2,5-diazabicyclo[2.2.1]hept-2-yl]ethanone;
- Compound No. 15: 1-[4-(4-Chloro-3-(trifluoromethyl)-phenyl)-3,6-dihydro-2H-pyridin-1-yl]-2-[5-(5-(trifluoromethyl)pyridin-2-yl)-2,5-diazabicyclo[2.2.1]-hept-2-yl]ethanone;
- Compound No. 16: 1-[4-(4-Chloro-3-(trifluoromethyl)-phenyl)-3,6-dihydro-2H-pyridin-1-yl]-2-(5-(pyridin-2-yl)-2,5-diazabicyclo[2.2.1]hept-2-yl)ethanone;
- Compound No. 17: 1-[4-(4-Chloro-3-(trifluoromethyl)-phenyl)-3,6-dihydro-2H-pyridin-1-yl]-2-[5-(5-fluoropyrimidin-2-yl)-2,5-diazabicyclo[2.2.1]hept-2-yl]-ethanone;
- Compound No. 18: 1-[4-(4-Chloro-3-(trifluoromethyl)-phenyl)-3,6-dihydro-2H-pyridin-1-yl]-2-[9-(5-(trifluoromethyl)pyridin-2-yl)-3,9-diazabicyclo[3.3.1]-non-3-yl]ethanone;
- Compound No. 19: 2-[8-(5-Fluoropyrimidin-2-yl)-3,8-diazabicyclo[3.2.1]oct-3-yl]-1-[4-(4-(trifluoromethyl)phenyl)-3,6-dihydro-2H-pyridin-1-yl]ethanone;
- Compound No. 20: 1-[4-(3-Chlorophenyl)-3,6-dihydro-2H-pyridin-1-yl]-2-[8-(5-fluoropyrimidin-2-yl)-3,8-diazabicyclo[3.2.1]oct-3-yl]ethanone;
- Compound No. 21: 2-[8-(5-Fluoropyrimidin-2-yl)-3,8-diazabicyclo[3.2.1]oct-3-yl]-1-[4-(3-(trifluoromethyl)phenyl)-3,6-dihydro-2H-pyridin-1-yl]ethanone;
- Compound No. 22: 1-[4-(4-Chlorophenyl)-3,6-dihydro-2H-pyridin-1-yl]-2-[8-(5-fluoropyrimidin-2-yl)-3,8-diazabicyclo[3.2.1]oct-3-yl]ethanone;
- Compound No. 23: 1-[4-(3,5-Bis(trifluoromethyl)-phenyl)-3,6-dihydro-2H-pyridin-1-yl]-2-[8-(5-fluoropyrimidin-2-yl)-3,8-diazabicyclo[3.2.1]oct-3-yl]-ethanone;
- Compound No. 24: 2-[8-(5-Fluoropyrimidin-2-yl)-3,8-diazabicyclo[3.2.1]oct-3-yl]-1-(4-(m-tolyl)-3,6-dihydro-2H-pyridin-1-yl)ethanone;
- Compound No. 25: 1-[4-(4-Chloro-3-(trifluoromethyl)-phenyl)-3,6-dihydro-2H-pyridin-1-yl]-2-[8-(pyrimidin-2-yl)-3,8-diazabicyclo[3.2.1]oct-3-yl]ethanone;
- Compound No. 26: 1-[4-(4-Chloro-3-(trifluoromethyl)-phenyl)-3,6-dihydro-2H-pyridin-1-yl]-2-[3-(5-fluoropyrimidin-2-yl)-3,8-diazabicyclo[3.2.1]oct-8-yl]-ethanone;
- Compound No. 27: 1-[4-(4-Chloro-3-(trifluoromethyl)-phenyl)-3,6-dihydro-2H-pyridin-1-yl]-2-(8-(pyridin-3-yl)-3,8-diazabicyclo[3.2.1]oct-3-yl)ethanone;
- Compound No. 28: Methyl ester of 6-(3-{2-[4-(4-chloro-3-(trifluoromethyl)phenyl)-3,6-dihydro-2H-pyridin-1-yl]-2-oxoethyl}-3,8-diazabicyclo[3.2.1]oct-8-yl)nicotinic acid;
- Compound No. 29: 6-(3-{2-[4-(4-Chloro-3-(trifluoromethyl)phenyl)-3,6-dihydro-2H-pyridin-1-yl]-2-oxoethyl}-3,8-diazabicyclo[3.2.1]oct-8-yl)nicotinic acid;
- Compound No. 30: 1-[4-(4-Chloro-3-(trifluoromethyl)-phenyl)-3,6-dihydro-2H-pyridin-1-yl]-2-[8-(6-(trifluoromethyl)pyridin-3-yl)-3,8-diazabicyclo[3.2.1]-oct-3-yl]ethanone;
- Compound No. 31: 2-[8-(5-Chloropyridin-2-yl)-3,8-diazabicyclo[3.2.1]oct-3-yl]-1-[4-(4-chloro-3-(trifluoromethyl)phenyl)-3,6-dihydro-2H-pyridin-1-yl]-ethanone;
- Compound No. 32: 1-[4-(4-Chloro-3-(trifluoromethyl)-phenyl)-3,6-dihydro-2H-pyridin-1-yl]-2-(8-(quinolin-2-yl)-3,8-diazabicyclo[3.2.1]oct-3-yl)ethanone;
- Compound No. 33: 1-[4-(4-Chloro-3-(trifluoromethyl)-phenyl)-3,6-dihydro-2H-pyridin-1-yl]-2-[8-(5-fluoropyridin-2-yl)-3,8-diazabicyclo[3.2.1]oct-3-yl]-ethanone;
- Compound No. 34: 2-[8-(6-Chloropyridin-3-yl)-3,8-diazabicyclo[3.2.1]oct-3-yl]-1-[4-(4-chloro-3-(trifluoromethyl)phenyl)-3,6-dihydro-2H-pyridin-1-yl]-ethanone;
- Compound No. 35: 1-[4-(3-(Trifluoromethyl)phenyl)-3,6-dihydro-2H-pyridin-1-yl]-2-[8-(3-(trifluoromethyl)pyridin-2-yl)-3,8-diazabicyclo[3.2.1]oct-3-yl]ethanone;
- Compound No. 36: Ethyl ester of 6-(3-{2-[4-(4-chloro-3-(trifluoromethyl)phenyl)-3,6-dihydro-2H-pyridin-1-yl]-2-oxoethyl}-3,8-diazabicyclo[3.2.1]oct-8-yl)-nicotinic acid;
- Compound No. 37: 2-(8-(Pyrazin-2-yl)-3,8-diazabicyclo[3.2.1]oct-3-yl)-1-[4-(3-(trifluoromethyl)-phenyl)-3,6-dihydro-2H-pyridin-1-yl]ethanone;
- Compound No. 38: 2-(8-(Pyrimidin-4-yl)-3,8-diazabicyclo[3.2.1]oct-3-yl)-1-[4-(3-(trifluoromethyl)-phenyl)-3,6-dihydro-2H-pyridin-1-yl]ethanone;
- Compound No. 39: 1-[4-(4-Chloro-3-(trifluoromethyl)-phenyl)-3,6-dihydro-2H-pyridin-1-yl]-2-(8-(pyrazin-2-yl)-3,8-diazabicyclo[3.2.1]oct-3-yl)ethanone;
- Compound No. 40: 2-(8-(Pyrazin-2-yl)-3,8-diazabicyclo[3.2.1]oct-3-yl)-1-(4-(m-tolyl)-3,6-dihydro-2H-pyridin-1-yl)ethanone;
- Compound No. 41: Methyl ester of 2-(3-{2-[4-(4-chloro-3-(trifluoromethyl)phenyl)-3,6-dihydro-2H-pyridin-1-yl]-2-oxoethyl}-3,8-diazabicyclo[3.2.1]oct-8-yl)pyrimidine-5-carboxylic acid;
- Compound No. 42: 2-(3-{2-[4-(4-Chloro-3-(trifluoromethyl)phenyl)-3,6-dihydro-2H-pyridin-1-yl]-2-oxoethyl}-3,8-diazabicyclo[3.2.1]oct-8-yl)pyrimidine-5-carboxylic acid;
- Compound No. 43: 1-[4-(4-Chloro-3-(trifluoromethyl)-phenyl)-3,6-dihydro-2H-pyridin-1-yl]-3-[8-(5-fluoropyrimidin-2-yl)-3,8-diazabicyclo[3.2.1]oct-3-yl]-propan-1-one;
- Compound No. 44: 1-[4-(4-Chloro-3-(trifluoromethyl)-phenyl)-3,6-dihydro-2H-pyridin-1-yl]-2-[5-(5-fluoropyrimidin-2-yl)-2,5-diazabicyclo[2.2.2]oct-2-yl]-ethanone;
- Compound No. 45: 2-[8-(5-fluoropyrimidin-2-yl)-3,8-diazabicyclo[3.2.1]oct-3-yl]-1-[4-(3-methoxyphenyl)-3,6-dihydro-2H-pyridin-1-yl]ethanone;
- Compound No. 46: 1-[4-(4-Chloro-3-(trifluoromethyl)-phenyl)-3,6-dihydro-2H-pyridin-1-yl]-2-[5-(6-(trifluoromethyl)pyridazin-3-yl)-2,5-diazabicyclo[2.2.1]-hept-2-yl]ethanone;
- Compound No. 47: 2-[8-(5-Fluoropyrimidin-2-yl)-3,8-diazabicyclo[3.2.1]oct-3-yl]-1-[3-(3-(trifluoromethyl)-4-chlorophenyl)-2,5-dihydropyrrol-1-yl]-ethanone;
- Compound No. 48: 6-(3-{2-[4-(4-Chloro-3-(trifluoromethyl)phenyl)-3,6-dihydro-2H-pyridin-1-yl]-2-oxoethyl}-3,8-diazabicyclo[3.2.1]oct-8-yl)nicotinamide;
- Compound No. 49: 2-[8-(5-Fluoropyrimidin-2-yl)-3,8-diazabicyclo[3.2.1]oct-3-yl]-1-[4-(2,3-dichlorophenyl)-3,6-dihydro-2H-pyridin-1-yl]ethanone;
- Compound No. 50: 1-[4-(4-Chloro-3-(trifluoromethyl)-phenyl)-3,6-dihydro-2H-pyridin-1-yl]-2-[8-(6-fluoropyridin-3-yl)-3,8-diazabicyclo[3.2.1]oct-3-yl]-ethanone;
- Compound No. 51: 2-[8-(5-Fluoropyrimidin-2-yl)-3,8-diazabicyclo[3.2.1]oct-3-yl]-1-[5-(3-(trifluoromethyl)phenyl)-3,6-dihydro-2H-pyridin-1-yl]ethanone;
- Compound No. 52: 2-[8-(5-Fluoropyrimidin-2-yl)-3,8-diazabicyclo[3.2.1]oct-3-yl]-1-[3-(3-(trifluoromethyl)phenyl)-2,5-dihydropyrrol-1-yl]ethanone;
- Compound No. 53: Methyl ester of 3-(1-{2-[8-(5-fluoropyrimidin-2-yl)-3,8-diazabicyclo[3.2.1]oct-3-yl]acetyl}-1,2,5,6-tetrahydropyridin-3-yl)benzoic acid;
- Compound No. 54: 2-[8-(5-Fluoropyrimidin-2-yl)-3,8-diazabicyclo[3.2.1]oct-3-yl]-1-[5-(2-(trifluoromethyl)phenyl)-3,6-dihydro-2H-pyridin-1-yl]ethanone;
- Compound No. 55: 1-[4-(4-Chloro-3-(trifluoromethyl)-phenyl)-3,6-dihydro-2H-pyridin-1-yl]-2-(8-(pyrimidin-5-yl)-3,8-diazabicyclo[3.2.1]oct-3-yl)ethanone;
- Compound No. 56: 2-[8-(5-Fluoropyrimidin-2-yl)-3,8-diazabicyclo[3.2.1]oct-3-yl]-1-[4-(3-(trifluoromethoxy)phenyl)-3,6-dihydro-2H-pyridin-1-yl]ethanone;
- Compound No. 57: 1-[4-(4-Chloro-3-(trifluoromethyl)-phenyl)-3,6-dihydro-2H-pyridin-1-yl]-2-[5-(6-(trifluoromethyl)pyridazin-3-yl)-2,5-diazabicyclo[2.2.2]-oct-2-yl]ethanone;
- Compound No. 58: N-[6-(3-{2-[4-(4-Chloro-3-(trifluoromethyl)phenyl)-3,6-dihydro-2H-pyridin-l-yl]-2-oxoethyl}-3,8-diazabicyclo[3.2.1]oct-8-yl)pyridin-3-yl]acetamide;
- Compound No. 59: 2-(8-(Quinolin-3-yl)-3,8-diazabicyclo[3.2.1]oct-3-yl)-1-[4-(3-trifluoromethyl-4-chlorophenyl)-3,6-dihydro-2H-pyridin-1-yl]ethanone;
in the form of the base or of an addition salt with an acid.

11. Process for the preparation of a compound of formula (I) according to any one of Claims 1 to 10, **characterized in that** a compound of formula (II): 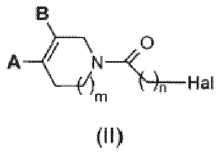 in which A, B, m and n are defined according to any one of Claims 1 to 10 and Hal represents a halogen atom, and a compound of general formula (III):
H-W-R5 (III)
in which W and R5 are defined according to any one of Claims 1 to 10,
are reacted.

12. Compound of formula (II): 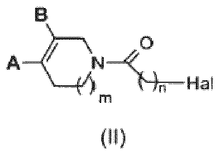 in which A, B, m and n are defined according to any one of Claims 1 to 10 and Hal represents a halogen atom; with the exception of 2-chloro-1-[4-(2-methoxyphenyl)-3,6-dihydro-2H-pyridin-1-yl]ethanone and 2-chloro-1-[4-(4-bromophenyl)-3,6-dihydro-2H-pyridin-1-yl]ethanone; in the form of the base or of an addition salt with an acid.

13. Medicament, **characterized in that** it comprises a compound of formula (I) according to any one of Claims 1 to 10 or an addition salt of this compound with a pharmaceutically acceptable acid.

14. Pharmaceutical composition, **characterized in that** it comprises a compound of formula (I) according to any one of Claims 1 to 10 or a pharmaceutically acceptable salt, and at least one pharmaceutically acceptable excipient.

15. Compound according to any one of Claims 1 to 10 for the preparation of a medicament intended for the prevention or treatment of central and peripheral neurodegenerative diseases, senile dementia, epilepsy, Alzheimer's disease, Parkinson's disease, Huntington's chorea, Down's syndrome, prion diseases, amnesia, schizophrenia, depression, bipolar disorder, amyotrophic lateral sclerosis, multiple sclerosis, cardiovascular conditions, post-ischaemic cardiac damage, cardiomyopathies, myocardial infarction, cardiac insufficiency, cardiac ischaemia, cerebral infarction, peripheral neuropathies, damage to the optic nerve and to the retina, retinal pigment degeneration, glaucoma, retinal ischaemia, macular degeneration, spinal cord traumas, cranial traumas, atherosclerosis, stenoses, cicatrization disorders, alopecia, cancers, tumours, metastases, leukaemias, respiratory disorders, pulmonary inflammation, allergy, asthma, chronic obstructive bronchopneumopathy, skin, somatic, visceral and neurological pain, chronic neuropathic and inflammatory pain, autoimmune diseases,
rheumatoid arthritis, ankylosing spondylarthritis, psoriatic arthritis, plaque psoriasis, bone fractures, bone diseases or osteoporosis.

16. Compound for the preparation of a medicament according to Claim 15 having the ability to inhibit the dimerization of the p75^{NTR} receptor independently of its ligand.

## Patentansprüche

1. Verbindung der Formel (I): 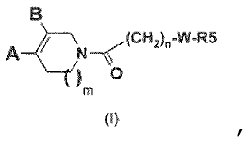 in der
- m 0 oder 1 bedeutet;
- A 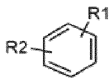 bedeutet
und B ein Wasserstoffatom bedeutet oder
A ein Wasserstoffatom bedeutet und B 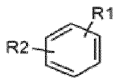 bedeutet;
- W- einen stickstoffhaltigen Heterocyclus aus der folgenden Reihe 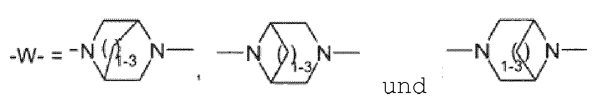 bedeutet,
- 1-3 1,2 oder 3 bedeutet;
- n 1 oder 2 bedeutet;
- R1 ein Halogenatom, eine (C₁-C₄)Alkylgruppe, einen Trifluormethylrest, eine (C₁-C₄)Alkoxygruppe oder einen Trifluormethoxyrest bedeutet;
- R2 ein Wasserstoffatom, ein Halogenatom, eine (C₁-C₄)Alkylgruppe, einen Trifluormethylrest, eine (C₁-C₄)Alkoxygruppe oder einen Trifluormethoxyrest, eine COOR-Gruppe oder eine CONH₂-Gruppe bedeutet;
- R5 eine Gruppe der Formel: 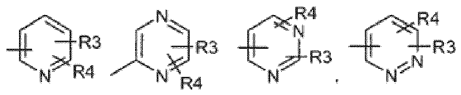 oder 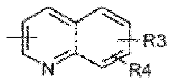 bedeutet,
- in denen R3 und R4, die in beliebigen verfügbaren Positionen liegen, unabhängig voneinander ein Wasserstoffatom, ein Halogenatom, eine (C₁-C₄)Alkylgruppe, eine (C₁-C₄)Alkoxygruppe, einen Trifluormethyl-, Trifluormethoxy- oder einen Cyanorest, eine COOH-, COO-Alkyl-, CONH₂-, CONR6R7- oder NHCOR-Gruppe bedeuten;
- R, R6 und R7 eine C₁-C₆-Alkylgruppe bedeuten;
als Base oder als Additionssalz mit einer Säure.

2. Verbindung nach Anspruch 1, in der R2 ein Wasserstoffatom, einen Trifluormethylrest, eine COOR-Gruppe oder eine CONH₂-Gruppe bedeutet, als Base oder als Additionssalz mit einer Säure.

3. Zusammensetzung nach Anspruch 1 oder 2, in der R5 eine Gruppe der Formel 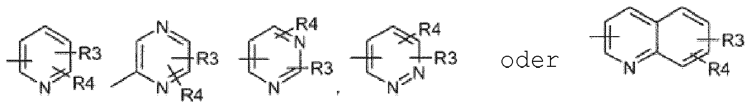 bedeutet,
worin R3 und R4 unabhängig voneinander ein Wasserstoffatom, ein Halogen, einen Trifluormethyl-, CONH₂-, COOH- oder NHCOCH₃-Rest bedeuten, als Base oder als Additionssalz mit einer Säure.

4. Verbindung nach Anspruch 1, in der:
- m 1 bedeutet;
- A 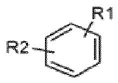 bedeutet
und B ein Wasserstoffatom bedeutet;
- W- einen stickstoffhaltigen Heterocyclus aus der Reihe: 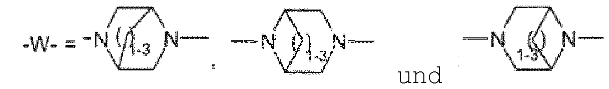 bedeutet;
- n 1 oder 2 bedeutet;
- R1 ein Halogenatom, eine (C₁-C₄)Alkylgruppe, einen Trifluormethylrest, eine (C₁-C₄)Alkoxygruppe oder einen Trifluormethoxyrest bedeutet;
- R2 ein Wasserstoffatom, ein Halogenatom, eine (C₁-C₄)Alkylgruppe, einen Trifluormethylrest, eine (C₁-C₄)Alkoxygruppe oder einen Trifluormethoxyrest bedeutet;
- R5 eine Gruppe der Formel 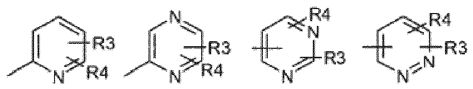
oder 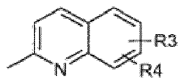 bedeutet,
in denen R3 und R4, die in beliebigen verfügbaren Positionen liegen, unabhängig voneinander ein Wasserstoffatom, ein Halogenatom, eine (C₁-C₄)Alkylgruppe, eine (C₁-C₄)Alkoxygruppe, einen Trifluormethyl-, Trifluormethoxy- oder Cyanorest, eine COOH- oder COO-Alkylgruppe bedeuten;
als Base oder als Additionssalz mit einer Säure.

5. Verbindung nach einem der vorhergehenden Ansprüche, in der W eine Gruppe der Formel aus der Reihe: 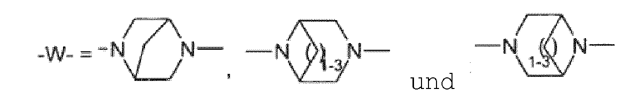 bedeutet,
als Base oder als Additionssalz mit einer Säure.

6. Verbindung nach einem der vorhergehenden Ansprüche, in der n = 1, als Base oder als Additionssalz mit einer Säure.

7. Verbindung nach einem der vorhergehenden Ansprüche, in der R1 ein Halogenatom oder einen Trifluormethylrest bedeutet, als Base oder als Additionssalz mit einer Säure.

8. Verbindung nach einem der vorhergehenden Ansprüche, in der R2 ein Wasserstoffatom oder einen Trifluormethylrest bedeutet, als Base oder als Additionssalz mit einer Säure.

9. Verbindung nach einem der vorhergehenden Ansprüche, in der R5 eine Gruppe der Formel 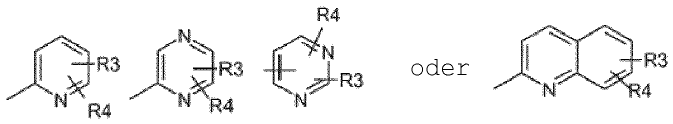 bedeutet,
worin R3 oder R4 unabhängig voneinander ein Wasserstoffatom, ein Halogenatom oder einen Trifluormethylrest bedeuten, als Base oder als Additionssalz mit einer Säure.

10. Verbindung nach einem der vorhergehenden Ansprüche, ausgewählt aus den folgenden Verbindungen:
- Verbindung Nr. 1: 1-[4-(3-Trifluormethylphenyl)-3,6-dihydro-2H-pyridin-1-yl]-2-[8-(5-trifluormethylpyridin-2-yl)-3,8-diazabicyclo[3.2.1]oct-3-yl]ethanon;
- Verbindung Nr. 2: 1-[4-(4-Chlorphenyl)-3,6-dihydro-2H-pyridin-1-yl]-2-(8-(pyrimidin-2-yl)-3,8-diazabicyclo[3.2.1]oct-3-yl)ethanon;
- Verbindung Nr. 3: 2-(3-(Pyrazin-2-yl)-3,8-diazabicyclo[3.2.1]oct-8-yl)-1-[4-(3-(trifluormethyl)phenyl)-3,6-dihydro-2H-pyridin-1-yl]ethanon;
- Verbindung Nr. 4: 2-(8-(Pyrimidin-2-yl)-3,8-diazabicyclo[3.2.1]oct-3-yl)-1-[4-(3-trifluormethylphenyl)-3,6-dihydro-2H-pyridin-1-yl]ethanon;
- Verbindung Nr. 5: 1-[4-(3-(Trifluormethyl)phenyl)-3,6-dihydro-2H-pyridin-1-yl]-2-[3-(5-trifluormethylpyridin-2-yl)-3,8-diazabicyclo[3.2.1]oct-8-yl]ethanon;
- Verbindung Nr. 6: 2-(8-(Pyridin-2-yl)-3,8-diazabicyclo[3.2.1]oct-3-yl)-1-[4-(3-trifluormethylphenyl)-3,6-dihydro-2H-pyridin-1-yl]ethanon;
- Verbindung Nr. 7: 1-[4-(4-Chlorphenyl)-3,6-dihydro-2H-pyridin-1-yl]-2-[8-(5-trifluormethylpyridin-2-yl)-3,8-diazabicyclo[3.2.1]oct-3-yl]ethanon;
- Verbindung Nr. 8: 1-[4-(4-Chlor-3-trifluormethylphenyl)-3,6-dihydro-2H-pyridin-1-yl]-2-[8-(5-trifluormethylpyridin-2-yl)-3,8-diazabicyclo[3.2.1]oct-3-yl]ethanon;
- Verbindung Nr. 9: 2-(8-(Chinolin-2-yl)-3,8-diazabicyclo[3.2.1]oct-3-yl)-1-[4-(3-trifluormethylphenyl)-3,6-dihydro-2H-pyridin-1-yl]ethanon;
- Verbindung Nr. 10: 1-[4-(4-Chlorphenyl)-3,6-dihydro-2H-pyridin-1-yl]-2-[3-(5-trifluormethylpyridin-2-yl)-3,8-diazabicyclo[3.2.1]oct-8-yl]ethanon;
- Verbindung Nr. 11: 1-[4-(4-Chlor-3-trifluormethylphenyl)-3,6-dihydro-2H-pyridin-1-yl]-2-[8-(5-fluorpyrimidin-2-yl)-3,8-diazabicyclo[3.2.1]oct-3-yl]ethanon;
- Verbindung Nr. 12: 2-[8-(5-Brompyrimidin-2-yl)-3,8-diazabicyclo[3.2.1]oct-3-yl]-1-[4-(4-chlor-3-trifluormethylphenyl)-3,6-dihydro-2H-pyridin-1-yl]-ethanon;
- Verbindung Nr. 13: 1-[4-(3-Trifluormethylphenyl)-3,6-dihydro-2H-pyridin-1-yl]-2-[5-(5-trifluormethylpyridin-2-yl)-2,5-diazabicyclo[2.2.1]hept-2-yl]ethanon;
- Verbindung Nr. 14: 1-[4-(4-Chlorphenyl)-3,6-dihydro-2H-pyridin-1-yl]-2-[5-(5-trifluormethylpyridin-2-yl)-2,5-diazabicyclo[2.2.1]hept-2-yl]ethanon;
- Verbindung Nr. 15: 1-[4-(4-Chlor-3-trifluormethylphenyl)-3,6-dihydro-2H-pyridin-1-yl]-2-[5-(5-trifluormethylpyridin-2-yl)-2,5-diazabicyclo[2.2.1]hept-2-yl]ethanon;
- Verbindung Nr. 16: 1-[4-(4-Chlor-3-trifluormethylphenyl)-3,6-dihydro-2H-pyridin-1-yl]-2-(5-pyridin-2-yl-2,5-diazabicyclo[2.2.1]hept-2-yl)ethanon;
- Verbindung Nr. 17: 1-[4-(4-Chlor-3-trifluormethylphenyl)-3,6-dihydro-2H-pyridin-1-yl]-2-[5-(5-fluorpyrimidin-2-yl)-2,5-diazabicyclo[2.2.1]hept-2-yl]ethanon;
- Verbindung Nr. 18: 1-[4-(4-Chlor-3-trifluormethylphenyl)-3,6-dihydro-2H-pyridin-1-yl]-2-[9-(5-trifluormethylpyridin-2-yl)-3,9-diazabicyclo[3.3.1]non-3-yl]ethanon;
- Verbindung Nr. 19: 2-[8-(5-Fluorpyrimidin-2-yl)-3,8-diazabicyclo[3.2.1]oct-3-yl]-1-[4-(4-trifluormethylphenyl)-3,6-dihydro-2H-pyridin-1-yl]ethanon;
- Verbindung Nr. 20: 1-[4-(3-Chlorphenyl)-3,6-dihydro-2H-pyridin-1-yl]-2-[8-(5-fluorpyrimidin-2-yl)-3,8-diazabicyclo[3.2.1]oct-3-yl]ethanon;
- Verbindung Nr. 21: 2-[8-(5-Fluorpyrimidin-2-yl)-3,8-diazabicyclo[3.2.1]oct-3-yl]-1-[4-(3-trifluormethylphenyl)-3,6-dihydro-2H-pyridin-1-yl]ethanon;
- Verbindung Nr. 22: 1-[4-(4-Chlorphenyl)-3,6-dihydro-2H-pyridin-1-yl]-2-[8-(5-fluorpyrimidin-2-yl)-3,8-diazabicyclo[3.2.1]oct-3-yl]ethanon;
- Verbindung Nr. 23: 1-[4-(3,5-Bistrifluormethylphenyl)-3,6-dihydro-2H-pyridin-1-yl]-2-[8-(5-fluorpyrimidin-2-yl)-3,8-diazabicyclo[3.2.1]oct-3-yl]ethanon;
- Verbindung Nr. 24: 2-[8-(5-Fluorpyrimidin-2-yl)-3,8-diazabicyclo[3.2.1]oct-3-yl]-1-(4-m-tolyl-3,6-dihydro-2H-pyridin-1-yl)ethanon;
- Verbindung Nr. 25: 1-[4-(4-Chlor-3-trifluormethylphenyl)-3,6-dihydro-2H-pyridin-1-yl]-2-[8-(pyrimidin-2-yl)-3,8-diazabicyclo[3.2.1]oct-3-yl]ethanon;
- Verbindung Nr. 26: 1-[4-(4-Chlor-3-trifluormethylphenyl)-3,6-dihydro-2H-pyridin-1-yl]-2-[3-(5-fluorpyrimidin-2-yl)-3,8-diazabicyclo[3.2.1]oct-8-yl]ethanon;
- Verbindung Nr. 27: 1-[4-(4-Chlor-3-trifluormethylphenyl)-3,6-dihydro-2H-pyridin-1-yl]-2-(8-pyridin-3-yl-3,8-diazabicyclo[3.2.1]oct-3-yl)ethanon;
- Verbindung Nr. 28: Methylester der 6-(3-{2-[4-(4-Chlor-3-trifluormethylphenyl)-3,6-dihydro-2H-pyridin-1-yl]-2-oxoethyl}-3,8-diazabicyclo[3.2.1]oct-8-yl)nikotinsäure;
- Verbindung Nr. 29: 6-(3-{2-[4-(4-Chlor-3-trifluormethylphenyl)-3,6-dihydro-2H-pyridin-1-yl]-2-oxoethyl}-3,8-diazabicyclo[3.2.1]oct-8-yl)nikotinsäure;
- Verbindung Nr. 30: 1-[4-(4-Chlor-3-trifluormethylphenyl)-3,6-dihydro-2H-pyridin-1-yl]-2-[8-(6-trifluormethylpyridin-3-yl)-3,8-diazabicyclo[3.2.1]oct-3-yl]ethanon;
- Verbindung Nr. 31: 2-[8-(5-Chlorpyridin-2-yl)-3,8-diazabicyclo[3.2.1]oct-3-yl]-1-[4-(4-chlor-3-trifluormethylphenyl)-3,6-dihydro-2H-pyridin-1-yl]ethanon;
- Verbindung Nr. 32: 1-[4-(4-Chlor-3-trifluormethylphenyl)-3,6-dihydro-2H-pyridin-1-yl]-2-(8-chinolin-2-yl-3,8-diazabicyclo[3.2.1]oct-3-yl)ethanon;
- Verbindung Nr. 33: 1-[4-(4-Chlor-3-trifluormethylphenyl)-3,6-dihydro-2H-pyridin-1-yl]-2-[8-(5-fluorpyridin-2-yl)-3,8-diazabicyclo[3.2.1]oct-3-yl]ethanon;
- Verbindung Nr. 34: 2-[8-(6-Chlorpyridin-3-yl)-3,8-diazabicyclo[3.2.1]oct-3-yl]-1-[4-(4-chlor-3-trifluormethylphenyl)-3,6-dihydro-2H-pyridin-1-yl]ethanon;
- Verbindung Nr. 35: 1-[4-(3-Trifluormethylphenyl)-3,6-dihydro-2H-pyridin-1-yl]-2-[8-(3-trifluormethylpyridin-2-yl)-3,8-diazabicyclo[3.2.1]oct-3-yl]ethanon;
- Verbindung Nr. 36: Ethylester der 6-(3-{2-[4-(4-Chlor-3-trifluormethylphenyl)-3,6-dihydro-2H-pyridin-1-yl]-2-oxoethyl}-3,8-diazabicyclo[3.2.1]oct-8-yl)nikotinsäure;
- Verbindung Nr. 37: 2-(8-Pyrazin-2-yl-3,8-diazabicyclo[3.2.1]oct-3-yl)-1-[4-(3-trifluormethylphenyl)-3,6-dihydro-2H-pyridin-1-yl]ethanon;
- Verbindung Nr. 38: 2-(8-Pyrimidin-4-yl-3,8-diazabicyclo[3.2.1]oct-3-yl)-1-[4-(3-trifluormethylphenyl)-3,6-dihydro-2H-pyridin-1-yl]ethanon;
- Verbindung Nr. 39: 1-[4-(4-Chlor-3-trifluormethylphenyl)-3,6-dihydro-2H-pyridin-1-yl]-2-(8-pyrazin-2-yl-3,8-diazabicyclo[3.2.1]oct-3-yl)ethanon;
- Verbindung Nr. 40: 2-(8-Pyrazin-2-yl-3,8-diazabicyclo[3.2.1]oct-3-yl)-1-(4-m-tolyl-3,6-dihydro-2H-pyridin-1-yl)ethanon;
- Verbindung Nr. 41: Methylester der 2-(3-{2-[4-(4-Chlor-3-trifluormethylphenyl)-3,6-dihydro-2H-pyridin-1-yl]-2-oxoethyl}-3,8-diazabicyclo[3.2.1]oct-8-yl)pyrimidin-5-carbonsäure;
- Verbindung Nr. 42: 2-(3-{2-[4-(4-Chlor-3-trifluormethylphenyl)-3,6-dihydro-2H-pyridin-1-yl]-2-oxoethyl}-3,8-diazabicyclo[3.2.1]oct-8-yl)pyrimidin-5-carbonsäure;
- Verbindung Nr. 43: 1-[4-(4-Chlor-3-trifluormethylphenyl)-3,6-dihydro-2H-pyridin-1-yl]-3-[8-(5-fluorpyrimidin-2-yl)-3,8-diazabicyclo[3.2.1]oct-3-yl]propan-l-on;
- Verbindung Nr. 44: 1-[4-(4-Chlor-3-trifluormethylphenyl)-3,6-dihydro-2H-pyridin-1-yl]-2-[5-(5-fluorpyrimidin-2-yl)-2,5-diazabicyclo[2.2.2]oct-2-yl]ethanon;
- Verbindung Nr. 45: 2-[8-(5-Fluorpyrimidin-2-yl)-3,8-diazabicyclo[3.2.1]oct-3-yl]-1-[4-(3-methoxyphenyl)-3,6-dihydro-2H-pyridin-1-yl]ethanon;
- Verbindung Nr. 46: 1-[4-(4-Chlor-3-trifluormethylphenyl)-3,6-dihydro-2H-pyridin-1-yl]-2-[5-(6-trifluorpyridazin-3-yl)-2,5-diazabicyclo[2.2.1]hept-2-yl]ethanon;
- Verbindung Nr. 47: 2-[8-(5-Fluorpyrimidin-2-yl)-3,8-diazabicyclo[3.2.1]oct-3-yl]-1-[3-(3-trifluormethyl-4-chlorphenyl)-2,5-dihydropyrrol-1-yl]ethanon;
- Verbindung Nr. 48: 6-(3-{2-[4-(4-Chlor-3-trifluormethylphenyl)-3,6-dihydro-2H-pyridin-1-yl]-2-oxoethyl}-3,8-diazabicyclo[3.2.1]oct-8-yl)nikotinamid;
- Verbindung Nr. 49: 2-[8-(5-Fluorpyrimidin-2-yl)-3,8-diazabicyclo[3.2.1]oct-3-yl]-1-[4-(2,3-dichlorphenyl)-3,6-dihydro-2H-pyridin-1-yl]ethanon;
- Verbindung Nr. 50: 1-[4-(4-Chlor-3-trifluormethylphenyl)-3,6-dihydro-2H-pyridin-1-yl]-2-[8-(6-fluorpyridin-3-yl)-3,8-diazabicyclo[3.2.1]oct-3-yl]ethanon;
- Verbindung Nr. 51: 2-[8-(5-Fluorpyrimidin-2-yl)-3,8-diazabicyclo[3.2.1]oct-3-yl]-1-[5-(3-trifluormethylphenyl)-3,6-dihydro-2H-pyridin-1-yl]ethanon;
- Verbindung Nr. 52: 2-[8-(5-Fluorpyrimidin-2-yl)-3,8-diazabicyclo[3.2.1]oct-3-yl]-1-[3-(3-trifluormethylphenyl)-2,5-dihydropyrrol-1-yl]ethanon;
- Verbindung Nr. 53: Methylester der 3-(1-{2-[8-(5-Fluorpyrimidin-2-yl)-3,8-diazabicyclo[3.2.1]oct-3-yl]acetyl}-1,2,5,6-tetrahydropyridin-3-yl)benzoesäure;
- Verbindung Nr. 54: 2-[8-(5-Fluorpyrimidin-2-yl)-3,8-diazabicyclo[3.2.1]oct-3-yl]-1-[5-(2-trifluormethylphenyl)-3,6-dihydro-2H-pyridin-1-yl]ethanon;
- Verbindung Nr. 55: 1-[4-(4-Chlor-3-trifluormethylphenyl)-3,6-dihydro-2H-pyridin-1-yl]-2-(8-pyrimidin-5-yl-3,8-diazabicyclo[3.2.1]oct-3-yl)ethanon;
- Verbindung Nr. 56: 2-[8-(5-Fluorpyrimidin-2-yl)-3,8-diazabicyclo[3.2.1]oct-3-yl]-1-[4-(3-trifluormethoxyphenyl)-3,6-dihydro-2H-pyridin-1-yl]ethanon;
- Verbindung Nr. 57: 1-[4-(4-Chlor-3-trifluormethylphenyl)-3,6-dihydro-2H-pyridin-1-yl]-2-[5-(6-trifluormethylpyridazin-3-yl)-2,5-diazabicyclo[2.2.2]oct-2-yl]ethanon;
- Verbindung Nr. 58: N-[6-(3{2-[4-(4-Chlor-3-trifluormethylphenyl)-3,6-dihydro-2H-pyridin-1-yl]-2-oxoethyl}-3,8-diazabicyclo[3.2.1]oct-8-yl)pyridin-3-yl]acetamid;
- Verbindung Nr. 59: 2-(8-Chinolin-3-yl-3,8-diazabicyclo[3.2.1]oct-3-yl)-1-[4-(3-trifluormethyl-4-chlorphenyl)-3,6-dihydro-2H-pyridin-1-yl]ethanon;
als Base oder als Additionssalz mit einer Säure.

11. Verfahren zur Herstellung einer Verbindung der Formel (I) nach einem der Ansprüche 1 bis 10, **dadurch gekennzeichnet, dass** man eine Verbindung der Formel (II): 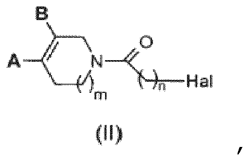 in der A, B, m und n gemäß einem der Ansprüche 1 bis 10 definiert sind und Hal ein Halogenatom bedeutet, und eine Verbindung der allgemeinen Formel (III),
H-W-R5 (III) ,
in der W und R5 gemäß einem der Ansprüche 1 bis 10 definiert sind, umsetzt.

12. Verbindung der Formel (II) 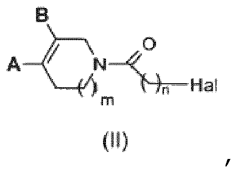 in der A, B, m und n gemäß einem der Ansprüche 1 bis 10 definiert sind und Hal ein Halogenatom bedeutet; mit Ausnahme von 2-Chlor-1-[4-(2-methoxyphenyl)-3,6-dihydro-2H-pyridin-1-yl]ethanon und von 2-Chlor-1-[4-(4-bromphenyl)-3,6-dihydro-2H-pyridin-1-yl]ethanon; als Base oder als Additionssalz mit einer Säure.

13. Arzneimittel, **dadurch gekennzeichnet, dass** es eine Verbindung der Formel (I) nach einem der Ansprüche 1 bis 10 oder ein Additionssalz dieser Verbindung mit einer pharmazeutisch unbedenklichen Säure umfasst.

14. Pharmazeutische Zusammensetzung, **dadurch gekennzeichnet, dass** sie eine Verbindung der Formel (I) nach einem der Ansprüche 1 bis 10 oder ein pharmazeutisch unbedenkliches Salz sowie mindestens einen pharmazeutisch unbedenklichen Grundstoff umfasst.

15. Verbindung nach einem der Ansprüche 1 bis 10 für die Herstellung eines Arzneimittels zur Vorbeugung oder Behandlung von degenerativen Erkrankungen des zentralen und peripheren Nervensystems, Altersdemenz, Epilepsie, Morbus Alzheimer, Morbus Parkinson, Huntington-Chorea, Down-Syndrom, Prionerkrankungen, Gedächtnisschwund, Schizophrenie, Depression, manisch-depressiver Psychose, amyotropher Lateralsklerose, multipler Sklerose, Herz-Kreislauf-Leiden, postischämischen Herzschäden, Kardiomyopathien, Myokardinfarkt, Herzinsuffizienz, Herzischämie, Hirninfarkt; peripheren Neuropathien, Schädigungen des Sehnervs und der Retina, Degeneration des Retinapigments, Glaukom, retinaler Ischämie, Makuladegeneration, Rückenmarks- und Schädeltraumen, Arteriosklerose, Stenosen, Vernarbungsleiden, Alopecia, Karzenomen, Tumoren, Metastasen, Leukämien, Erkrankungen der Atemwege, Lungenentzündung, Allergie, Asthma, chronischobstruktiver Bronchopneumopathie, Haut-, Körper-, Eingeweide- und Nervenschmerz, chronischen neuropathischen und entzündlichen Schmerzen, Autoimmunerkrankungen, rheumatoider Polyarthritis, ankylosierender Spondylarthritis, Psoriasis-Rheumatismus, Plaque-Psoriasis, Knochenbrüchen, Knochenerkrankungen oder Osteoporose.

16. Verbindung für die Herstellung eines Arzneimittels nach Anspruch 15 mit der Fähigkeit, die Dimerisierung des p75^{NTR}-Rezeptors unabhängig von seinem Liganden zu hemmen.
